(19) **Europäisches Patentamt / European Patent Office / Office européen des brevets**

(11) **EP 0 931 551 B1**

(12) **EUROPEAN PATENT SPECIFICATION**

(45) Date of publication and mention
of the grant of the patent:
**04.04.2007 Bulletin 2007/14**

(51) Int Cl.:
*A61K 39/395* (2006.01)  *C07K 16/28* (2006.01)
*A61K 35/12* (2006.01)  *C12N 5/06* (2006.01)
*G01N 33/569* (2006.01)

(21) Application number: **97122186.6**

(22) Date of filing: **16.12.1997**

(54) **Method of producing therapeutics for transmissible spongiform encephalopathy and non-infective blood and tissue derived products as well as methods of producing these**

Verfahren zur Herstellung von Mittel zur Therapie der übertragbaren Spongiformen Enzephalopathie und nicht-infektiösen Blut- und Gewebeprodukten sowie die hergestellten Produkte

Méthode pour produir des thérapeutiques pour l'Encéphalopathie Spongiforme transmissible, produits sanguins et tissulaires non-infectieux et procédés pour les obtenir

(84) Designated Contracting States:
**AT BE CH DE DK ES FI FR GB GR IE IT LI LU MC NL PT SE**

(43) Date of publication of application:
**28.07.1999 Bulletin 1999/30**

(60) Divisional application:
**01123316.0**
**01123317.8**
**01127424.8 / 1 214 943**
**01127425.5 / 1 215 497**

(73) Proprietor: **UNIVERSITY OF ZURICH**
**8600 Zürich (CH)**

(72) Inventors:
• **Aguzzi, Adriano**
**8001 Zürich (CH)**
• **Klein, Michael A.**
**53925 Kall (DE)**
• **Raeber, Alex**
**8057 Zürich (CH)**
• **Weissmann, Charles**
**8053 Zürich (CH)**
• **Zinkernagel, Rolf**
**8126 Zumikon (CH)**

(74) Representative: **Modiano, Micaela Nadia**
**Modiano Josif Pisanty & Staub Ltd**
**Thierschstrasse 11**
**80538 München (DE)**

(56) References cited:
**WO-A-89/12458**

• **BENDHEIM ET AL.: "Nearly ubiquitous tissue distribution of the scrapie agent precursor protein" NEUROLOGY, vol. 42, January 1992, pages 149-156, XP002065592**
• **O'ROURKE ET AL.: "SCID mouse spleen does not support scrapie agent replication" JOURNAL OF GENERAL VIROLOGY, vol. 75, 1994, pages 1511-1514, XP002066607**
• **CASHMAN ET AL.: "Cellular isoform of the scrapie agent protein participates in lymphocyte activation" CELL, vol. 61, 6 April 1990, pages 185-192, XP002066608**
• **LASM¹ZAS ET AL.: "Immune system-dependent and -independent replication of the scrapie agent" JOURNAL OF VIROLOGY, vol. 70, no. 2, February 1996, pages 1292-1295, XP002066609**
• **EKLUND ET AL.: "Pathogenesis of scrapie virus infection in the mouse" THE JOURNAL OF INFECTIOUS DISEASES, vol. 117, 1967, pages 15-22, XP002065593**
• **BLÄTTLER ET AL.: "PrP-expressing tissue required for transfer of scrapie infectivity from spleen to brain" NATURE, vol. 389, 4 September 1997, page 69-73 XP002065594**
• **BÜELER ET AL.: "Normal development and behaviour of mice lacking the neuronal cell-surface PrP protein" NATURE, vol. 356, 16 April 1992, pages 577-582, XP002065595**
• **HUNT ET AL.: "Monoclonal antibody LR1 recognizes murine heat-stable antigen, a marker of antigen-presenting cells and developing hematopoietic cells" INTERNATIONAL ARCHIVES OF ALLERGY AND IMMUNOLOGY, vol. 111, no. 3, November 1996, pages 218-229, XP002065597**

- ERMAK ET AL.: "Depletion and repopulation lymphocytes in Peyer's patches of mice after total lymphoid irradiation" LABORATORY INVESTIGATION, vol. 59, no. 5, November 1988, pages 591-597, XP002065598
- ASSENSI ET AL.: "In vivo influence of the anti-B220 monoclonal antibody administration of the T cell differentiation in mice" MICROBIOLOGY AND IMMUNOLOGY, vol. 33, no. 4, 1989, pages 329-339, XP002065599
- KLEIN ET AL.: "A crucial role for B cells in neuroinvasive scrapie" NATURE , vol. 390, 16 - 18 December 1997, pages 687-690, XP002065601
- DENIS ET AL.: "T cells in hypersensititivity pneumonitis: effects of in vivo depletion of T cells in a mouse model" AMERICAN JOURNAL OF RESPIRATORY CELL AND MOLECULAR BIOLOGY, vol. 6, no. 2, February 1992, pages 183-189, XP002081282
- BUTTKE ET AL.: "Positive selection of mouse B and T lymphocytes and analysis of isolated populations by flow cytometry" JOURNAL OF IMMUNOLOGICAL METHODS, vol. 58, no. 1-2, 11 March 1983, pages 193-207, XP002081283
- BERTOLINI ET AL.: "A new 'two step' procedure for 4.5 log depletion of T and B cells in allogeneic Transplantation and of neoplastic cells in autologous transplantation" BONE MARROW TRANSPLANTATION, vol. 19, no. 6, March 1996, page 615-619 XP002081284
- KITAMURA ET AL.: "A B cell-deficient mouse by targeted disruption of the membrane exon of the immununoglobulin $\mu$ chain gene" NATURE, vol. 350, 4 April 1991, page 423-426 XP002065596
- MOMBAERTS ET AL.: "RAG-1-Deficient Mice have no mature B and T lymphocytes" CELL, vol. 68, 6 March 1992, pages 869-877, XP002081285
- SHINKAI ET AL.: "RAG-2-deficient mice lack mature lymphocytes owing to the inability to intitiate V(D)J rearrangement" CELL, vol. 68, 6 March 1992, pages 855-867, XP000604771
- KIMBERLIN ET AL.: "Pathogenesis of mouse scrapie: Dynamics of agent replication in spleen, spinal cord and brain after infection by different routes" JOURNAL OF COMPARITIVE PATHOLOGY, vol. 89, no. 4, 4 October 1979, pages 551-562, XP002081286
- MILLSON ET AL.: "Early distribution of radioactive liposomes and scrapie infectivity in mouse tissues following administration by different routes" VETERINARY MICROBIOLOGY, vol. 4, no. 2, 1979, pages 89-99, XP002081287
- DIOMEDE L ET AL: "Activation effects of a prion protein fragment [PrP-(106-126)] on human leucocytes." BIOCHEMICAL JOURNAL, (1996 DEC 1) 320 ( PT 2) 563-70. JOURNAL CODE: 9YO. ISSN: 0264-6021., XP002081288 ENGLAND: United Kingdom
- CAUGHEY B ET AL: "Detection of prion protein mRNA in normal and scrapie -infected tissues and cell lines." JOURNAL OF GENERAL VIROLOGY, (1988 MAR) 69 ( PT 3) 711-6. JOURNAL CODE: I9B. ISSN: 0022-1317., XP002081289 ENGLAND: United Kingdom
- FIELD E J ET AL: "Cellular sensitization in kuru, Jakob-Creutzfeldt disease and multiple sclerosis: with a note on the biohazards of slow infection work." ACTA NEUROLOGICA SCANDINAVICA, (1975 APR) 51 (4) 299-309. JOURNAL CODE: 1BS. ISSN: 0001-6314., XP002081290 Denmark
- MAYER R J ET AL: "The role of protein ubiquitination in neurodegenerative disease." ACTA BIOLOGICA HUNGARICA, (1991) 42 (1-3) 21-6. REF: 24 JOURNAL CODE: 0DF. ISSN: 0236-5383., XP002081291 Hungary
- FOLEY G L ET AL: "Rabies-induced spongiform change and encephalitis in a heifer." VETERINARY PATHOLOGY, (1995 MAY) 32 (3) 309-11. JOURNAL CODE: XBQ. ISSN: 0300-9858., XP002081292 United States
- OUTRAM ET AL: 'Reduced Susceptibility to Scrapie in Mice after Steroid Administration', NATURE, vol.249, 28 June 1974, pages 855-856 XP009025783
- BERGER M.L.: 'Humoral and Cell-Mediated Immune Mechanisms in the Production of Pathology in Avirulent Semliki Forest Virus Encephalitis', INFECTION and IMMUNITY, vol.30, October 1980, pages 244-253 XP002112384

**Description**

The present invention relates to the use of selective B-cell depletants for manufacturing a medicament for the treatment or prevention of transmissible spongiform encephalopathy. Further, the invention relates to non-infective human TSE-free body fluid products and to human TSE-free tissue derived products and to suitable methods for the manufacture thereof.

## Background art

Transmissible spongiform encephalopathies (TSE's) comprise a group of slow degenerative diseases of the CNS such as Creutzfeld-Jakob disease (CJD), new variant CJD (termed vCJD)[1,2], Gerstmann-Sträussler-Scheinker disease (GSS) and kuru in man and scrapie in sheep or BSE (mad cow disease) in cattle.

The occurrence of these exotic illnesses is still fortunately very low, probably occuring at 1:1,000,000 but there are striking similarities when compared to the Alzheimer-syndrome. However, BSE is now reported to have reached epidemic proportions in England and is caused by the use of rendered materials in cattle feed and can originate with scrapie infected sheep. Dairy cattle in particular are at the highest measurable risk. A tragically similar incidence has occurred with humans.

During the production of human growth hormone from human glands collected from cadavers, the pathogenic agent of Creutzfeld-Jacob Syndrome was introduced. Several Cases have now been reported in patients treated with this growth hormone. The patients were predominantly children, whereas the disease normally attacks adults over 50 years of age.

These examples point out the potential danger of these new diseases and the difficulties in diagnosing and treating them effectively.

The unusual properties of the pathogenic agent, designated as "prion" (Prusiner, S.B. Novel proteinaceous infectious particles cause scrapie. *Science* 216, 136-144. (1982)) include the extremely long incubation periods, exceeding one year, and resistance to high temperatures, formaldehyde treatment and UV irradiation (Gordon, W.S. *Vet Rec* 58,516 (1946); Pattison, I.H. Resistance of the scrapie agent to formalin. *J comp Pathol* 75, 159-164 (1965); Alper et al., The exceptionally small size of the scrapie agent. *Biochem. Biophys. Res. Commun.* 22, 278-284 (1966); Latarjet et al., Inactivation of the scrapie agent by near monochromatic ultraviolet light. *Nature* 227, 1341-1343 (1970)) Speculations arose early on that the scrapie agent might be devoid of nucleic acid (Alper et al., Does the agent of scrapie replicate without nucleic acid? *Nature* 214, 764-766 (1967); Gibbon, R.A. and Hunter, G.D. Nature of the scrapie agent. *Nature* 215, 1041-1043 (1967); Pattison, I.H. and Jones, K.M The possible nature of the transmissible agent of scrapie. *Vet. Rec.* 80, 2-9 (1967)). Considerable evidence now supports the "protein only" hybothesis (Prusiner, S.B. and Hsiao, K.K. Human prion diseases. *Ann. Neurol.* 35, 385-395 (1994); Weissmann, C. Molecular biology of prion diseases. *Trends Cell Biol.* 4, 10-14 (1994)) which proposes that the prion is devoid of nucleic acid and identical with PrP$^{Se}$, a modified form of PrP$^C$. PrP$^C$ is a normal host protein (Oesch et al., A cellular gene encodes scrapie PrP 27-30 Protein. *Cell* 40, 735-746 (1985); Chesebro et al., Identification of scrapie prion protein-specific mRNA in scrapie-infected and uninfected brain. *Nature* 315, 331-333 (1985) found predominantly on the outer surface of neurons, but also in many other tissues (Manson et al., The prion protein gene: a role in mouse embryogenesis? *Development* 115, 117-122 (1992); Bendheim et al., Nearly ubiquitous tissue distribution of the scrapie agent precursor protein. *Neurology* 42, 149-156 (1992)). PrP$^{Se}$ is defined as a protease-resistant form of PrP$^C$ which readily forms aggregates after detergent treatment (Mc Kinley et al., Scrapie prion rod formation in vitro requires both detergent extraction and limited proteolysis: *J. Vitrol.* 65, 1340-1351 (1991)). No chemical differences have so far been detected between PrP$^{Se}$ and PrP$^C$ (Stahl et al., Structural studies of the scrapie prion protein using mass spectrometry and amino acid sequencing. Biochemistry 32, 1991-2002 (1993)). Prusiner proposed that PrP$^{Se}$, when introduced into a normal cell, causes the conversion of PrP$^C$ or its precursor into PrP$^C$ (Oesch et al., Search for a scrapie-specific nucleic acid: a progress report. *Ciba. Found. Symp.* 135, 209-223 (1988); Prusiner et al., Transgenetic studies implicate interactions between homologous PrP isoforms in scrapie prion replication. *Cell* 63, 673-686 (1990); Bolton, D.C. and Bendheim, P.E. A modified host protein model of scrapie. *Ciba. Found. Symp.* 135, 164-181 (1988)). The conversion is believed to result from a conformational rearrangement of PrP$^C$. Some researchers still adhere to the virino hypothesis which holds that the infectious agent consists of a nucleic acid genome and the host-derived PrP, which is recruited as some sort of coat (Dickinson, A.G. and Outram, G.W. Genetic aspects of unconventional virus infections: the basis of the virino hypothesis. *Ciba. Found. Symp.* 135, 63-83 (1988); Hope; J. The nature of the scrapie agent: the evolution of the virino. *Ann. N. Y. Acad. Sci.* 724, 282-289 (1994)). Finally, the possibility that the infectious agent is a virus with unusual properties is still upheld by some (Diringer et al., The nature of the scrapie agent: the virus theory. *Ann. N. Y. Acad. Sci.* 724, 240-258 (1994); Pocchiari, M. Prions and related neurological diseases. *Molec. Aspects. Med.* 15, 195-291 (1994); Rohwer, R.G. The scrapie agent: "a virus by any other name". *Curr. Top. Microbiol. Immunol.* 172, 195-232 (1991)). No credible evidence for the existence of a scrapie-specific nucleic acid, as demanded by the virus and the virino hyptohesis, has yet been forthcoming (Oesch et al., vide supra; Kellings et al., Futher analysis of nucleic acids in purified scrapie prion preparations by improved return refocusing gel electrophoresis. *J. Gen. Virol.* 73, 1025-1029 (1992)).

Prusiner and his colleagues were the first to purify PrP$^{Se}$ and demonstrate physical linkage to scrapie infectivity (Bolton et al., Identification of a protein thath purifies with the scrapie prion. *Science* 218, 1309-1311 (1982)). A collaboration between the groups of Prusiner, Hood and Weissmann led to the isolation of PrP cDNA and to the realization that PrP$^C$ was a normal host protein and that PrP$^{Se}$ was an isoform of PrP$^C$ (Oesch et al., vide supra (1985)). Weissmann and his collaborators (Basler et al., Scrapie and cellular PrP isoforms are encoded by the same chromosomal gene. *Cell* 46, 417-428 (1986)) cloned the PrP gene *(Prn-P)* and Prusiner's group showed the linkage between genetic susceptibility to prion disease and the *Prn-p* gene in mouse (Prusiner et al., vide supra (1990)) and man (Hsiao et al., Linkage of a prion protein missense variant to Gerstmann-Straussler syndrome. *Nature* 338, 342-345 (1989)). Several groups reported physical data supporting conformational differences between PrP$^C$ and PrP$^{Se}$ (Caughey et al., Secondary structure analysis of the scrapie-associated protein PrP 27-30 in water by infrared spectroscopy. *Biochemistry* 30, 7672-7680 (1991); Cohen et al., Structural clues to prion replication. *Science* 264, 530-531 (1994); Huang et al., Proposed three-dimensional structure for the cellular prion protein. *Proc. Natl. Acad. Sci. U.S.A.* 91, 7139-7143 (1994); Pan et al., Conversion of alpha-helices into beta-sheets features in the formation of the scrapie prion proteins. *Proc. Natl. Acad. Sci. U.S.A.* (1993); Safar et al., Conformational transitions, dissociation, and unfolding of scrapie amyloid (prion) protein. *J. Biol. Chem.* 268, 20276-20284 (1993)).

Since there is no reliable marker of transmissible spongiform encephalopathy infectivity, the kinetics of replication of the infectious agent cannot be studied specifically since the physical carriers of prions are not known. However, an increasing body of evidence from early experiments and from recent studies points to the importance of two distinct phases of replication during the life cycle of the prion, the infectious agent causing spongiform encephalopathies. In the first phase, replication of infectivity is thought to take place primarily in lymphoid organs (Eklund et al., Pathogenesis of scrapie virus infection in the mouse. *J. infect.* Dis. 117, 15-22 (1967); Clarke, M.C. and Komberlin, R.H. Pathogenesis of mouse scrapie: distribution of agent in the pulp and stroma of infected spleens. *Vet. Microbial.* 9, 215-225 (1984); Fraser, H. and Dickinson, A.G. Studies of the lymphoreticular system in the pathogenesis of scrapie: the role of spleen and thymus. *J. Comp. Pathol.* 88, 563-573 (1978)). For example, infectivity can be demonstrated in the spleen as early as 4 days after *i.p.* or *i.c.* infection. This is true even if infection takes place via the intracerebral route (Kimberlin, R.H. and walker, C.A. Pathogenesis of experminetal scrapie. *Ciba. Found. Symp.* 135, 37-62 (1988)), and replication of the infectious agent in the spleen precedes intracerebral replication even if infectivity is.administered intracerebrally (Rubenstein et al., Scrapie-infected spleen: analysis of infectivity, scrapie-associated fibrils, and protease-resistant proteins. *J. infect. Dis.* 164, 29-35 (1991)). Infectivity can also accumulate in other components of the lymphoreticular system (LRS), e.g. in lymph nodes and in Peyer's plaques of the small intestine, where replication of infectivity can be demonstrated almost immediately following oral administration of prion preparations. The rapid establishment of a plateau of the infectious titer in the spleen at a relatively early time point during the latency time suggests that the availability of prion repication sites is rate-limiting in the LRS. It is not known however, whether this plateau is due to a limited number of spleen cells supporting prion replication, or rather to limited availability of prion replication sites within each cell. The nature of the cells supporting prion replication within the LRS is uncertain. Indirect evidence obtained by studies in which the spleen was removed at variable intervals *after i.p.* infection suggests that the critical tissue compartment is long-lived and does not consist primarily of lymphocytes. In addition, ablation of lymphocytes by total body irradiation does not seem to affect the incubation time of mouse scrapie (Fraser et al., The scrapie disease process is unaffected by ionising radiation. *Prog. Clin. Biol. Res.* 317, 653-658 (1989)). Taken together, these and other findings suggest that follicular dendritic cells (FDC) may be the main population of cells involved in LRS replication of prions. Indeed, PrP accumulates in FDCs in the spleen of wild-type and nude mice, and *i.p.* infection does not lead to cerebral scrapie in SCID mice (whose FDCs are thought of be functionally impaired) while it efficiently provokes the disease in nude mice which bear a selective T-cell defect (Muramoto et al., Species barrier prevents an abnormal isoform of prion protein from accumulating in follicular dendritic cells of mice with Creutzfeldt-Jakob disease. *J. Virol.* 67, 6808-6810 (1993)).

Though above delineated steps are thought to be important in the natural history of transmissible spongiform encephalopathy within an infected organism, the limiting factor or physical entity involved in the development and spread of transmissible spongiform encephalopathy after peripheral infection, that is to say the physical carrier of the prion, is still not known. Even though precise monitoring of the epidemic spread of transmissible spongiform encephalopathy is rendered extremely difficult by the long incubation times involved (up to 30 years), it appears to be likely that peripheral infection, e.g. by alimentary exposure, is the most relevant route of propagation. Any attempt to combat transmissible spongiform encephalopathy should thus focus on such limiting factors or physical entities involved in the development of the disease after periferal infection. However, detailed knowledge about such limiting factor(s) or entities is an essential prerequisite to the design of improved therapeutic approaches aimed at interfering with prion replication and spread within an infected victim. Though a first therapeutic approach based on the administration of prednisolone as immunosuppressant has been recently proposed by Aguzzi et al. in <u>The Lancet</u> **350**: 1519-1520 (1997), the treatment proposed is relatively crude and should be regarded as provisional since it affects many cell types in addition to the unknown limiting factors and physical entity likely to be directly involved in prion spread and replication.

Further, knowledge about the identity of the physical carriers of prions would allow the design of improved assay methods for determining the infectivity of potentially infective materials like blood products or tissue derived products and for an improved monitoring of the epidemic progress of transmissible spongiform encephalopathy within infected populations. Also, knowledge about the interaction of the physical carriers of prions with further physical entities involved in pathogenesis would allow the monitoring of the disease progress within an infected victim and/or the verification of the effectivity of therapeutic treatment.

Still further, once the identity of the physical carriers is known, suitable methods for the separation of said physical carriers of prions from body fluid or tissue derived products intended for medical use or industrial application may be tailored on demand.

Accordingly, there is an urgent need for the specific identification of the limiting factors and physical entities in the development of spongiform encephalopathy after peripheral infection. There is further a need in providing improved medicaments for combating spongiform encephalopathy in infected organisms, that is to say humans and animals. Still further, there is a need in providing improved assay methods for the diagnosis and/or monitoring of the progress or regress of transmissible spongiform encephalopathy in infected organisms or in organisms suspected of being infected. Such assay methods are also needed for the safety testing of body fluid or tissue derived products derived from such organisms. Still further, there is a need in providing body fluid or tissue derived products which are not infective in order to prevent the further spread of transmissible spongiform encephalopathy within the infected human and animal populations. Still further there is a need in providing a method for the manufacture of such uninfective body fluid or tissue derived products. Still further there is a need in proving suitable reagents being capable of recognizing the crucial physical entity involved in the spread of spongiform encephalopathy.

Satisfaction of above needs as well as of further needs which will become apparent hereinafter is an object of the present invention.

## Summary of the invention

In order to meet above objects and to satisfy above needs, in one embodiment, the present invention provides the use of selective B-cell depletants for the manufacture of a medicament for the treatment or prevention of transmissible spongiform encephalopathy in infected humans or animals.

In a further embodiment, the present invention provides a human TSE-free body fluid or tissue derived product, characterized in that it has been depleted from B-cells in vitro, wherein the B-cells are depleted by means of a medicament comprising a selective B-cell depletant.

In a further embodiment, the present invention provides a method for the manufacture of a human TSE-free body fluid or tissue derived product, characterized in that said method comprises a step of separating B-cells from said body fluid or tissue derived product.

In a further embodiment, the present invention provides a method for the manufacture of a human TSE-free body fluid or tissue derived product, characterized in that said body fluid or tissue derived product is isolated from non-living humans that are only B-cell deficient.

In a further embodiment, the present invention provides the use of an antibody directed against infective B-cells.

Further embodiments of the present invention are set out in the dependent claims.

## Detailed description of the invention

The present invention involves detailed investigations about the nature of the limiting factors and/or physical entities in the development of spongiform encelophalopathy after periferal infection. Thus, the present invention involves identification of the physical carriers of prions and of the mechanisms involved in the spread of infectivity.

### Definitions

As referred to in the present application, B-cells (or B--lymphocytes) are to be understood as members of a subset of lymphocytic cells which are precursors of plasma cells which produce antibodies; they are able to recognize free antigens and antigens located on cells. As referred to in the present application, T-cells (or T-lymphocytes) are to be understood as members of a subset of lymphocytic cells responsible for cellular immunity and the production of immunomodulating substances. As referred to in the present application, the term 'lymphocytes' designates cells which participate in the humoral and cell-mediated immune defense, and which accordingly comprise B-cells and T-cells. As referred to in the present application, the term 'animals' encompasses all eukaryotic organisms excluding plants. The reference to T-cell and related products and methods is retained in the present disclosure and figures merely for illustrative and comparative purposes. Similarly the reference to assay methods capable of monitoring or diagnosing the spread of transmissible spongiform encephalopathy is retained for merely illustrative purposes.

Figures

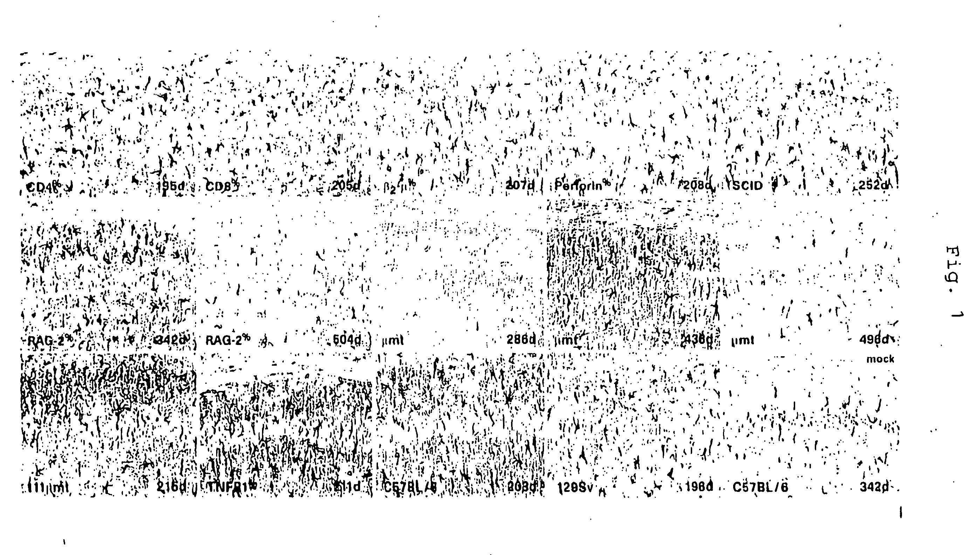

Figure 1 shows the brain histopathology of immune deficient and control mice after i.p. inoculation of scrapie prions. The hippocampal formation was immunostained for glial fibrillary acidic protein, and identical segments of the pyramidal cell ribbon were microphotgraphed (200x). Intense, diffuse gliosis was visible in brains of T-cell-deficient, SCID, TNF-r1$^{0/0}$, t11 $\mu$MT, and infected control mice. Some rag-2$^{0/0}$ and $\mu$MT mice showed spongiform encephalopathy, but others of the same genotype did not display any pathology after similar time periods following i.p. inoculation, and were indistinguishable from mock-infected C57BL/6 mice.

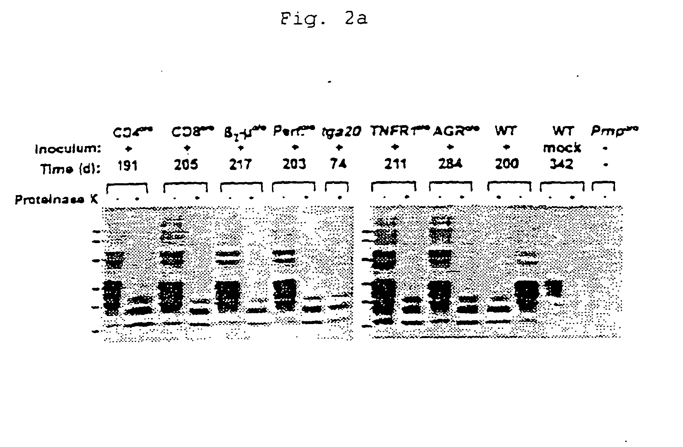

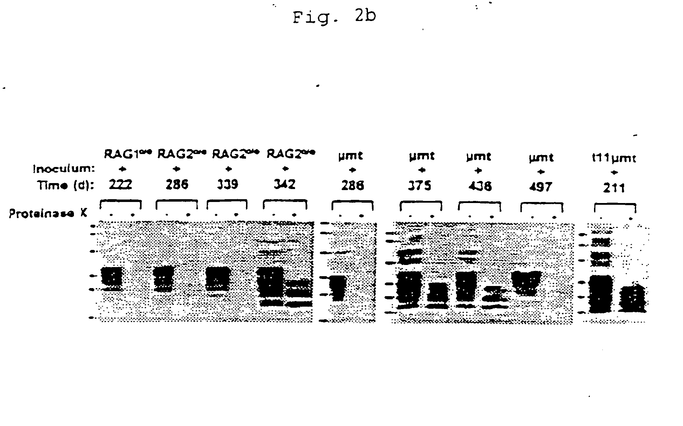

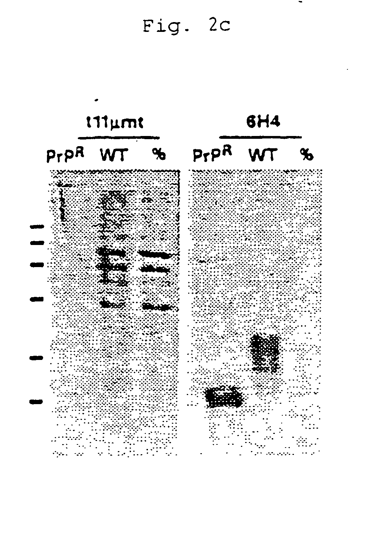

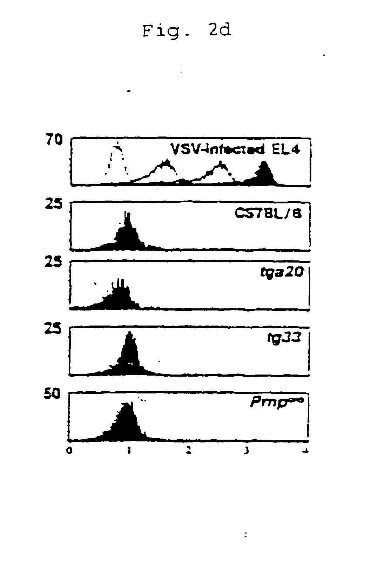

Figure 2 relates to the Western blot analysis of brains of immune-deficient mice after i.p. inoculation with transmissible spongiform encephalopathy prions and lack of specific antibodies against PrP in t11$\mu$MT mice. Figs. a,b are Western blots of brain material electrophoresed native (-) or after digestion with proteinase K (PK) (+). Large amounts of PK-resistant prion protein (PrP$^{SC}$) were detected in all mice that had developed spongiform encephalopathy, as well as in one agr$^{0/0}$ (a) two rag-2$^{0/0}$ and two $\mu$MT mice (b). One further B-cell-deficient mouse proved negative for PrP$^{SC}$ (not shown), and no clinical symptoms of spongiform encephalopathy were detected in any B-cell-deficient mice irrespective of accumulation of PrP$^{SC}$. **Fig. c** shows a Western blot prepared with recombinant murine PrP from E. coli (PrP$^{R}$), total brain protein extract from a wild-type mouse (WT), and total brain protein extract from a Prnp$^{0/0}$ mouse (0/0)[15]. Blots were incubated with serum from a t11$\mu$MT mouse inoculated with prions i.p. (left), stripped and reprobed with monoclonal antibody 6H4 to recombinant PrP (right). The presence of PrP-specific antibodies, as indicated by a 20K band in lane PrP$^{R}$ and by a cluster of bands present in lane WT but absent from lane 0/0, is evidence with 6H4 antibody but undetectable in t11$\mu$MT serum. Relative molecular mass markers (top to bottom): 105K, 82K, 45K, 37.3K, 28.6K, 19.4K. **Fig. d** shows the FACS analysis of immunoreactivity of t11$\mu$MT serum. Ordinate: cell counts; abscissa: logarithm of fluorescence intensity. Serum from a t11$\mu$MT mouse 210 days after i.p. inoculation with prions was diluted 1:10 and 1:100, stained VSV-infected EL4 cells (top panel, unfilled area) almost as strongly as VSV-specific monoclonal antibody VI24 (filed area). In contrast, immunoreaction of t11$\mu$MT serum (1:10) with CD3$^{+}$ T-cells from C57HL/6, tga20, tg33 (ref.29) and Prnp$^{0/0}$ mice (lower panels) did not exceed background, like normal C57BL/6 serum on EL4 cells (top panel, dotted line). The same profiles were obtained when probes were stained with serum of untreated t11 $\mu$MT mice (data not shown).

**Figure3a an 3b** display a flow analysis printout showing enriched B-cell and T-cell populations.

**Figure 4** Shows the infectivity of splenocytes in Wild type mice and Spleen mice.

Figures 5a-5c Show the infectivity in different cell types of Wild type mice, T-cell mice and Spleen mice.

Investigations carried out by the inventors

The role of the B-cells

As apparent from the prior art, the development of neurological disease after peripheral infection with transmissible spongiform encephalopathy depends on abnormal prion expansion within the cells of the lymphoreticular system [3,4]. The skilled man is however aware that the immune system comprises several components whose identity and precise function and specific interaction with the remaining components are still the object of extensive scientific investigation. The inventors have investigated for the first time the roles of different components of the immune system by using a panel of immune-defecent mice inoculated with prions intraperitoneally and found that defects effecting only T-cells had no apparent effect, but that all mutations that disrupted the differentiation and response of B-cells prevented the development of clinical spongiform encephalopathy. As an absence of B-cells and of antibodies correlates with severe defects in follicular dendritic cells, a lack of any of these three components may prevent the development of clinical spongiform encephalopathy. The key function of the follicular dendritic cells has been postulated inter alia by Muramoto, vide supra. However, the inventors found surprisingly that spongiform encephalopathy developed after peripheral inoculation in mice expressing immunoglobulins that were exclusively of the M subclass and without detectable specificity for the normal form of the prion PrP$^{c}$, and in mice which had B-cells but no functional follicular dendritic cells. Thus, the inventors have found out that differentiated B-cells are crucial for neuroinvasion by spongiform encephalopathy, regardless of the specificity of their receptors.

The effect of combined immune defects on the pathogenesis of spongiform encephalopathy was studied in mice deficient in *rag-2* (ref.5) and *rag-1* (ref.6), which lack B- and T-cells, in *scid* (severe combined immune deficient) mice, and in *agr*$^{0/0}$ mice, which lack rag-2 as well as the receptors for interferon-$\alpha/\beta$[7] and interferon-$\gamma$[8]. Such mice were obtained according to methods well-known in the art of genetic engineering. For controls, inbred mice of strains C57BL/6 and 129/Sv which are the genetic backgrounds of all other mouse strains used were inoculated as well. To investigate the role of T-cells, the inventors used mice with targeted disruption of the genes encoding CD4 (ref. 9), CD8 (ref. 10), $\beta_2$-microglobulin[11] or perforin[12]. Selective depletion of B-cells was studied in $\mu$MT mice[13], which have a targeted disruption of the transmembrane exon of the immunoglobulin u-chain gene, do not produce any immunoglobulins and suffer from a B-cell differentiation block at the large-to small pre-B-cell transition, yet bear complete and functional T-cell subsets.

After intracerebral (i.c.) challenge with prions, all immune-deficient mice developed clinical symptoms of spongiform encephalopathy. This was confirmed by histopathological analysis (not shown) and by transmission of disease to indicator *tga*20 mice, which over-express the normal prion protein (PrP$^C$) and are hypersensitive to spongiform encephalopathy[14] (Table 1). Transmission to Prnp$^{0/0}$ mice[15], which do not express PrP$^c$ and are resistant to spongiform encephalopathy[16] (n=4), did not induce disease after >210 days, as expected for bona fide spongiform encephalopathy. In all groups, latency times from inoculation to first appearance of clinical symptoms and to terminal disease (Table 2), as well as brain prion infectivity titres (Table 1), were similar to those of control mice.

Thus, if prions where delivered to the central nervous system, spongiform encephalopathy pathogenesis and prion expansion in the brain proceeded without any detectable influence of the immune status of the host.

When mice were exposed to prions through the intraperitoneal (i.p.) route, mice homozygous-null for CD4, CD8, $\beta_2$-microglobulin or perforin developed the initial symptoms of disease and terminal spongiform encephalopathy with latency periods similar to those of C57BL/6 and 129/Sv mice (Table 2), and reached analogous prion titres in both spleen and brain (Table 1). Thus the inventors concluded that CD8$^+$ cytotoxic and CD4$^-$ helper T-cells are not rate-limiting for spongiform encephalopathy after peripheral inoculation of prions, in agreement with the observation that nude mice develop spongiform encephalopathy normally after i.p. inoculation[3].

In contrast, no disease appeared after i.p. inoculation in $\mu$MT and in rag-deficient (*rag-1*$^{o/o}$, *rag-2*$^{o/o}$ and *agr*$^{o/o}$) mice, and no prion infectivity was detectable in their spleens (Table 1). In SCID C57BL/6 Mice, disease was marginally prolonged, which disagrees with earlier results[4,17] and may be due to incomplete immune deficiency of SCID mice in specific genetic backgrounds[13.], because SCID C.B-17 mice (whose immune defect is less leaky) did not develop disease (Table 2). B-cell differentiation to immune competence exhibits redundancy at many points; that renders such cells only partially sensitive to genetic manipulation.

Histopathological examination of brain sections revealed generalized spongiform encephalopathy in all wild-type and immune-deficient mice clinically diagnosed as spongiform encephalopathy-sick (Fig. 1). In addition, and despite lack of clinical symptoms, spongiform encephalopathy was seen in 1/7 *rag*-deficient and 1/6 $\mu$MT mice (at random sampling) 342 and 436 days after i.p. inoculation (Fig. 1), and significant prion titres were found in brains of 3/7 *rag*-deficient mice and 1/3 $\mu$MT mice (Table 1). Western blot analysis revealed accumulation of the disease form of prion, PrP$^{SC}$, in the brains of 2/6 rag -deficient and 2/6 $\mu$MT mice inoculated i.p. (Fig. 2). The remaining rag-deficient and $\mu$MT mice did not accumulate PrP$^{SC}$ as late as 504 days after inoculation.

For the sake of absolute scrutiny, it may be concluded that the latter findings are compatible with incipient spongiform encephalopathy in a minor fraction of B-cell-deficient mice. Therefore, although it prevents 'neuroinvasion' of the spongiform encephalopathy agent in most cases, absence of B-cells uncovers a slower, <50% efficient mechanism of pathogenesis which may cause spongiform encephalopathy in situations of immune deficiency. It should be emphazised that even then, B-cell deficiency prolongs the delay between PrP$^{SC}$ accumulation, onset of spongiform encephalopathy histopathology and clinical symptoms beyond the typical life expectancy of mice.

These results suggest that B-cells may 'transport' prions from lymphoid organs to nervous tissue. Alternatively, the apparent protection of B-cell-deficient mice from prions administered i.p. may result from the absence of immunoglobulins. Complexing of PrP$^{SC}$ with antibodies may favour nucleation (a process proposed to underlie the formation of prion infectivity[20]) or may opsonize PrP$^{SC}$ and enhance access to lymphoid sites of abnormal prion expansion. It also may suggest that animals become more able to propagate infection if the genetic change is later in B-cell development. To clarify this question, the inventors inoculated t11$\mu$MT mice ($\mu$MT mice expressing a rearranged IgM transgene directed against the glycoprotein of vesicular stromatitis virus) and found that they could support normal B-cell differentiation but exclusively expressed the transgenic IgM heavy chain, had a heavily skewed and very limited antibody repertoire, and lacked immunoglobulins of the D, G, E and A subclasses. Such mice were obtained according to methods well-known in the art.

After i.p. inoculation with prions, t11$\mu$MT mice developed disease with a latency comparable to that of wild-type mice (Table 2) and accumulated PrP$^{SC}$ in their brains (Fig. 2b). Serum from both uninfected and terminally spongiform encephalopathy-sick t11MT mice inoculated i.p. was shown by western blotting and by flow-assisted cell sorting (FACS) analysis not to crossreact with PrP$^C$ (Fig. 2c, d), suggesting that IgGs are not the effectors of prion 'neuroinvasion', and that a specific humoral immune response (at least as assessed by FACS and western-blot analysis) cannot be correlated with peripheral pathogenesis of spongiform encephalopathy. However, for the sake of absolute scrutiny, one cannot exclude the possibility that IgMs below the threshold of detectability, or indirect effects of antibodies, may be involved in spongiform encephalopathy pathogenesis. This corresponds to the difficulty in obtaining reliable disease transmission from soluble serum components from diseased animals.

B-cells are required for maturation of follicular dendritic cells (FDCs) and formation of germinal centres. Protection of B-cell-deficient mice may therefore result from the absence of FDCs, especially as FDs accumulate PrP$^{SC}$ extensively in i.p.-inoculated mice[3] and in the tonsils of patients suffering from new variant CJD[21]. Thus, the inventors inoculated mice lacking tumour-necrosis factor receptor-1 (TNF-R1$^{0/0}$)[22], which have virtually no germinal centres in lymphatic organs and very few, if any, FDCs[23], despite differentiation of functional B- and T-cells. These mice developed spongiform encephalopathy after both i.c. and i.p. inoculation, as did control mice (Table 2), thus disproving a prime role for FDCs in peripheral pathogenesis and supporting the inventors previous results that adoptive transfer of fetal liver cells (which does not efficiently replace FDCs[24]) can restore high spleen prion titres after i.p. inoculation[25].

EP 0 931 551 B1

TABEL 1

**Table 1 Scrapie symptoms, scrapie histopathology and infectivity titres in immunodeficient mice**

| Genotype | Primary infection (route of inoculation: i.c.) | | | Infectivity bioassay | |
|---|---|---|---|---|---|
| | Incubation (d) | Symptoms | Pathology | Brain | Spleen |
| CD-4$^{o/o}$ | 176 | + | + | 7.2* (65 d ± 2†) | 6.6 (71 d = 2) |
| CD-4$^{o/o}$ | 154 | + | + | 7.4 (63 d = 1) | 7 (67 d = 2) |
| scid | 167 | + | + | 7.3 (64 d ± 1) | 5.5 (81 d ± 2) |
| scid | 167 | + | + | 7.6 (62 d ± 2) | 5.2 (83 d ± 1) |
| rag-2$^{o/o}$ | 171 | + | + | 7.3 (64.5 d ± 2) | <0 (>200 d) |
| agr$^{o/o}$ | 182 | + | + | 7.3 (64.5 d ± 1) | <0 (>145 d) |
| μMT | 175 | + | + | 7.9 (59 d ± 5) | <0 (>200 d) |

| | Primary infection (route of inoculation: i.p.) | | | Infectivity bioassay | |
|---|---|---|---|---|---|
| CD-4$^{c/o}$ | 191 | + | + | 7.3 (64 d ± 11) | ND |
| CD-4$^{o/o}$ | 195 | + | + | 7.5 (62.5 d ± 2) | ND |
| scid | 214 | + | + | 7.3 (64 d ± 1) | 5.2 (83 d ± 1) |
| scid | 249 | + | + | 7.7 (61 d ± 2) | 5 (85 d ± 1) |
| rag-2$^{o/o}$ | 286 | – | + | 6.5 (72 d ± 2) | <0 (>200 d) |
| rag-2$^{o/o}$ | 286 | – | – | <0 (>200 d) | <0 (>200 d) |
| rag-2$^{o/o}$ | 339 | – | – | <1 (>122 d) | <2 (>115 d) |
| rag-2$^{o/o}$ | 342 | – | + | 7.5 (65 d ± 1) | <2 (>115 d) |
| rag-1$^{c/o}$ | 222 | – | – | <1 (>122 d) | <2 (>115 d) |
| agr$^{o}$ | 284 | – | + | 7.2 (65 d ± 0) | <0 (>139 d) |
| agr$^{o/o}$ | 349 | – | – | <0 (>139 d) | <0 (>139 d) |
| μMT | 286 | – | – | <0 (>200 d) | <0 (>200 d) |
| μMT | 286 | – | – | <0 (>200 d) | <0 (>200 d) |
| μMT | 375 | – | – | 7.8 (60 d ± 1) | <0 (>200 d) |
| μMT | 436 | – | + | ND | ND |
| TNF-R1$^{o/o}$ | 211 | + | + | 7.7 (61 d ± 1) | ND |
| TNF-R1$^{o/o}$ | 212 | + | + | 7.7 (60 d ± 1) | ND |

For infectivity bioassays, brain or spleen homogenates were injected intracerebrally into groups of four tga20 mice. ND, not determined.
* prion titres expressed as log LD$_{50}$ per g of spleen or brain tissue.
† Incubation time, in days, of indicator tga20 mice (average ± standard deviation).

TABEL 2

Table 2 Latency of scrapie in different immunodeficient mice

| Defect | Genotype | Intracerebral route | | Intraperitoneal route | |
|---|---|---|---|---|---|
| | | Scrapie | Time to terminal disease (d) | Scrapie | Time to terminal disease (d) |
| T | CD-4$^{0/0}$* | 7/7 | 159 ± 11 | 8/8 | 191 ± 1 |
| T | CD-8$^{0/0}$* | 6/6 | 157 ± 15 | 6/6 | 202 ± 5 |
| T | $\beta_2$-$\mu^{0/0}$* | 8/8 | 162 ± 11 | 7/7 | 211 ± 6 |
| T | Perforin$^{0/0}$* | 3/4† | 171 ± 2 | 4/4 | 204 ± 3 |
| T and B | SCID* | 7/8† | 160 ± 11 | 6/8† | 226 ± 15 |
| T and B | SCID§ | 4/4 | 152 ± 1 | 1/4‖ | 289 |
| T and B | rag-2$^{0/0}$* | 7/7 | 167 ± 2 | 0/7 | Healthy (>504) |
| T and B | rag-1$^{0/0}$* | 3/3 | 175 ± 2 | 0/5 | Healthy (>258) |
| T and B | agr$^{0/0}$¶ | 6/6 | 184 ± 10 | 0/7 | Healthy (>450) |
| B | μMT* | 8/8 | 181 ± 6 | 0/8 | Healthy (>534) |
| IgG | t11μMT* | 5/5 | 170 ± 3 | 4/4 | 223 ± 2 |
| FDC | TNF-R1$^{0/0}$# | 7/7 | 165 ± 3 | 9/9 | 216 ± 4 |
| Controls | 129Sv | 4/4 | 167 ± 9 | 4/4 | 193 ± 3 |
| | C57BL/6 | 4/4 | 166 ± 2 | 4/4 | 206 ± 2 |

All mice developed spongiform encephalopathy after i.c. inoculation. In contrast, B-cell-deficient mice stayed healthy after i.p. inoculation of RML scrapie prions.
* Genetic background was inbred C57BL/6.
† One Perforin$^{0/0}$ and one SCID mouse suffered from intercurrent death 135 and 141 days after inoculation, respectively.
‡ Two SCID C57BL/6 mice remained healthy and were killed 303 and 323 days after inoculation.
§ Genetic background was inbred C.B-17.
‖ 3/4 SCID C.B-17 mice remained healthy (>340 d). Four further SCID C.B-17 mice were challenged with 100 μl of a 10⁻¹ dilution of RML prions, and all remained healthy (>340 d).
¶ Genetic background was C57BL/6 × 129Sv.
# Genetic background was inbred 129Sv.

Thus, the inventors have identified B-cells and B-cell-dependent processes as a limiting factor in the development of transmissible spongiform encephalopathy after peripheral infection. Accodingly, the present invention provides a novel, specific and therefore more preferable procedure to suppress that component of the immune system which is responsible for the prion spread, namely the B-cells.

The role of the T-cells

The role of T-cell in the present disclosure is reported merely to better understand the role of the B-cells in the present invention.
Still further, the inventors have studied the role of B-cell-dependent processes during pathogenesis. Accordingly, the inventors have carried out further experiments aiming at establishing the amount and nature of possible interaction of infective B-cells with the remaining components of the immune system, e.g. with T-cells. Results of the inquiry about such interaction and design of suitable therapeutic measures influencing such interaction are a further aspect influencing the present invention.

As pointed out above, it is known that mice devoid of functional PrP genes (Prn-p$^{0/0}$) are resistant to transmissible spongiform encephalopathy and do not propagate prions (Büeler et al. Cell, 73, 1339-1347, 1993). Thus, reintroduction of PrP transgenes into Prn-p$^{0/0}$ should restore transmissible spongiform encephalopathy. Departing from this concept, the inventors conducted studies in Prn-p% mice transgenic for PrP genes controlled by tissue specific promotors. Such mice may be obtained by the man skilled in genetic engineering according to methods well-known in the art. Specifically, the inventors used 'T-cell mice' (Ick promotor; Chaffin et al., EMBO J. 9, 3821-3829) which express PrP exclusively in T-cells and 'spleen mice' (IRF-1 promoter/Eu enhancer; Yamada et al., Proc.Natl.Acad-Sci.USA. 88, 532-536, 1991) which express PrP in splenocytes and at low level in brain. Challenge of spleen-mice with prions led to the development of spongiform encephalopathy in that spleen mice succumbed at a late stage due to brain disease and showed propagation of prions in spleen and thymus as well as in brain. On the other hand, T-cell mice showed no propagation of prions. Accordingly, these results are fully consistent with the prior experiments as described hereinabove and confirm the crucial role of B-cells (Table 3).

TABLE 3

| Mice | Incubation time Mean Days ± sd | n/no |
|---|---|---|
| Prn-p$^{+/+}$ | 196±4 | 10/10 |
| Prn-P$^{0/0}$ | >500 | 0/3 |
| "Spleen mice" | 263±5 | 7/13 |
| "T cell mice" | >500 | 0/5 |

Table 1: Transmission of mouse prions to transgenic mice with ectopic PrP expression

To further investigate the role of B-cell dependent processes, in a second step, the infectivity of the splenocytes from spleen mice was selectively determined in a bioassay. It was found that, though most of the infectivity was indeed carried by the B-cells, the T-cells were also contaminated to some extent (Table 4).

TABLE 4

| Cell Fraction | Titer (LD50 units/10$^a$ cells) |
|---|---|
| splenocytes | ~200 |
| B cells | ~500 |
| T cells | ~100 |
| non-B, non-T cells | <1 |

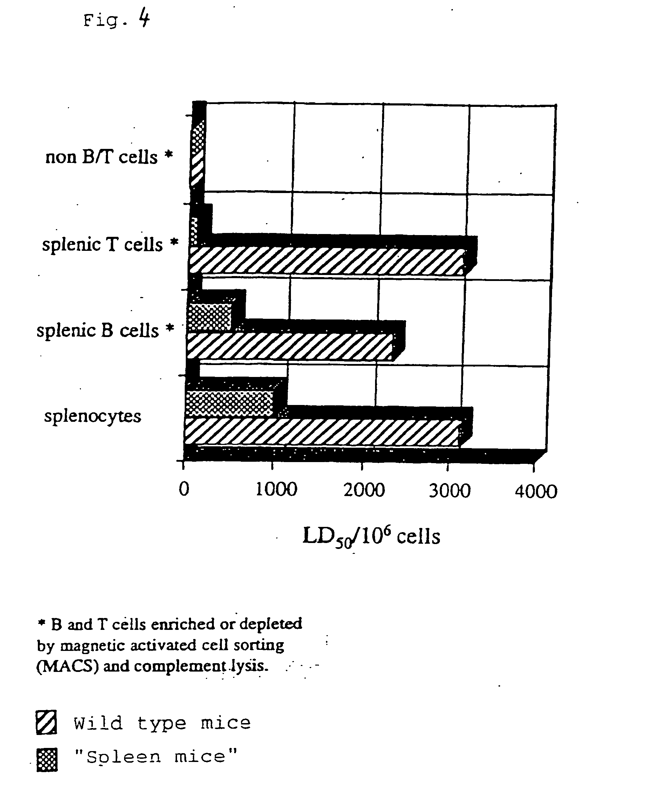

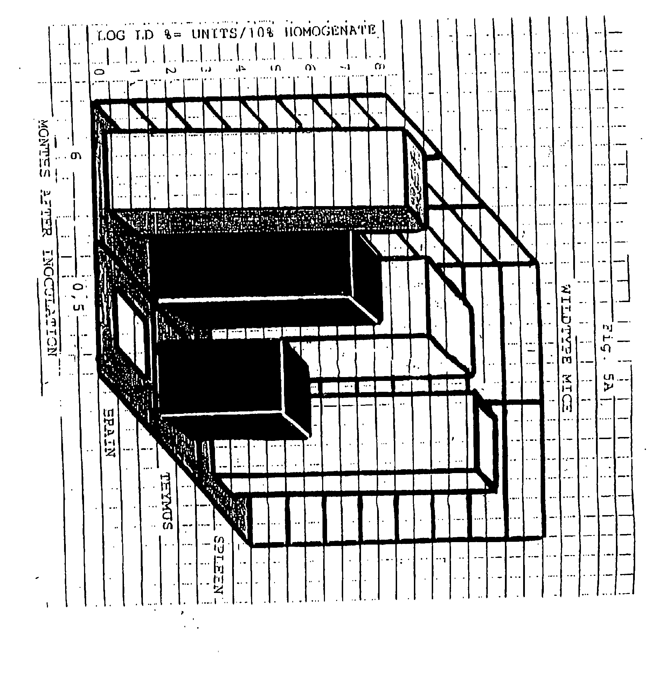

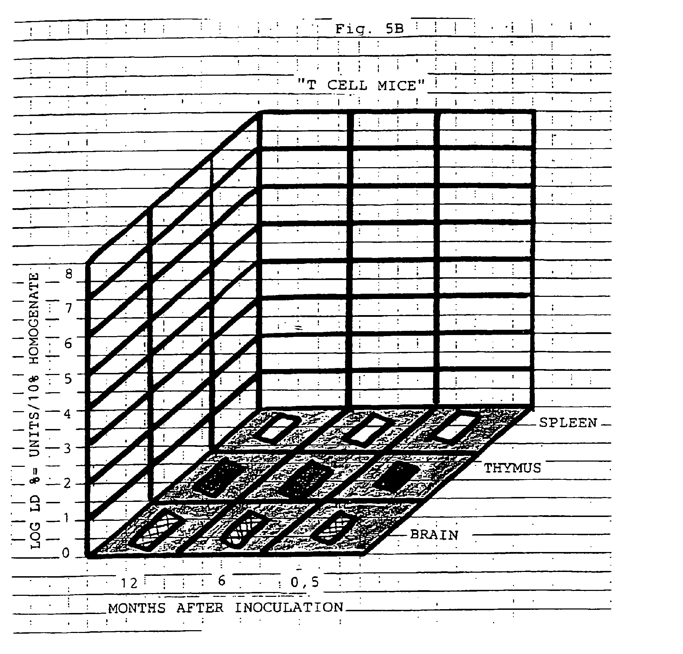

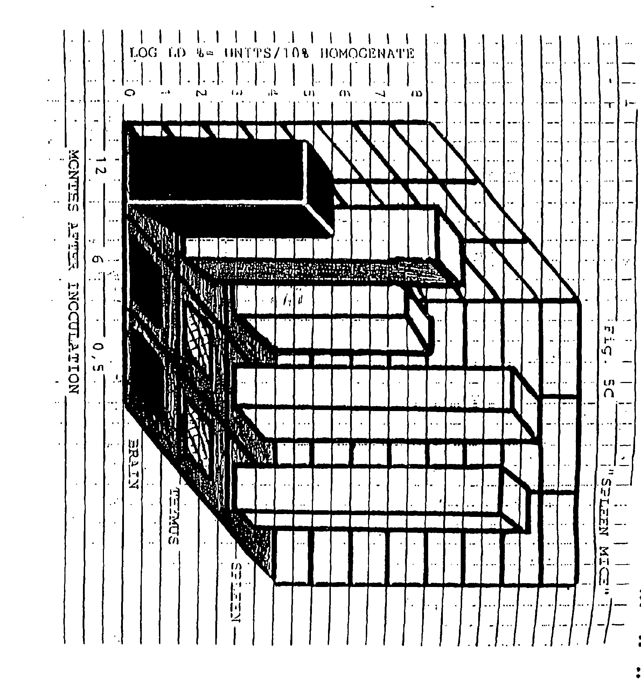

Table 2: Infectivity of total and fractionated splenocytes from "Spleen mice" 120 days after i.p. inoculation with prions. cells were fractionated by magnetic activated cell sorting (MACS) using anti-B220 antibodies for B cells and anti-Thy 1.2 antibodies for T-cells.
This newly found contamination shown by the T-cells of the spleen mice seems to be in contradiction with the fact that the T-cells of T-cell mice do not show any contamination (see Figures 4 and 5). However, what initially seems to be a contradiction (infectivity of T-cells in some cases, non-infectivity of T-cells in other cases), in reality implies and supports the existence of an interaction between the B-cells (the carriers of infectivity) and the T-cells (which, as such, are not able to propagate infectivity). Spleen mice contain both T-cells and B-cells, and upon infection of the B-cells, a B-cell mediated secondary infection of the spleen mice T-cells takes place. On the contrary, T-cell mice do not contain B-cells, and as a consequence of this lack of infectivity carriers, the T-cell mice T-cells are not subject to infection.

Conclusions

As pointed out above, not only the crucial carrier of infectivity, namely the B-cells, has been identified, but also a powerful tool for the monitoring of the spread of transmissible spongiform encephalopathy within the immune system of an infected human or animal has been provided for the first time by the inventors. Indeed, the present invention allows the distinction between the occurence of infected B-cells alone and the further occurence of secondarily infected T-cells.

In particular, above finding that removal of B-lymphocytes by surface antigen B-cell autolysis limits or prevents the transmission of prion disease infection demonstrates that the absence of such cellular components prevents transfer of infectivity to other cells, such as T-cells, or development of disease. It follows logically that B-cells are predictive of the pathological outcome and progression of prion disease. These disease specific components of the cellular immune system can then effectively stage developing disease or predict the status of disease in an individual organisim undergoing treatment.

Lymphocytes are isolated from blood by standard techniques known to preserve phenotypic cellar features. Cells isolated in this manner may be evaluated without manipulation or fixed by suitable methods and then introduced into liquids solutions composed of well know constituents containing binding partners or antibodies characteristic for cells that may express "prion disease" phenotypic determinants or classical lymphocyte determinants distributed among progenitor and/or daughter cells of a given devolpmental lineage in way characteristic of the disease. These components may be selected from but not limited to cellular diferentitation, CD, antigens such as CD 19, CD 20, etc and/or binding partners specific for certain intracellular or extra cellular disease specific cellular phenotypes such as antibodies to normal or abnormal prion proteins. This components may be disease strain or species specific. These phenotypes or distribution of phenotypes correlate with the infectivity or the transmission of infectivity. It is to be realized that such CD or prion disease specific antigens or determinants may be differentially distributed in qualitative or quantitative manner among lymphocytes of different stages of development and functional lineage's. The relationship of such phenotypic determinants in cell populations is diagnostic of the presence of disease, the presence of disease progress in advancing or the degree of regression of disease undergoing treatment, depending on the status of the organism in question.

Since the unusual and novel observation that B-cells provide the necessary germinal site for the disease promulgation, the analysis of specific determinants in B- and T-lymphocytes will provide insight into the disease progression. It is to be understood that the means of detecting these proportional relationships of cells among different phenotypic populations could be achieved by means of histopathological methods or automated flow cytometric methods utilizing sophisticated data analysis algorithms to display results in a readily interpretable way.

Further aspects and preferred embodiments of the invention

Still further, according to the present invention, selective suppression of infective B-cells can be accomplished by treatment with an adequate amount of antibody to an infective B-cell surface marker. One should anticipate that examining unusual dispositions of B-cells or T-cells or of their progenitors and products may be important. Preferably, this antibody recognizes the infective B-cell and not the stem cell, thus allowing for a later repopulation of B-cells by the stem cell. Preferably, procedures well known in the art may help in the preparation of such antibodies. Accordingly, the use of such antibodies in a medicament comprising such an antibody are a further aspect contemplated by the present invention.

An further object of the present invention is the use of selective B cell depletants for the manufacture of a medicament for the treatment of transmissible spongiform encephalopathy in infected humans or animals. A "B-cell depletant" as referred to in the present application is a reagent or a kit of reagents which upon administration either alone, together or sequentially leads to depletion of B-cells in the organism being treated. Any B-cell depletant known in the art may be used to achieve the above stated object of the present invention. Suitable B-cell depletants comprise either immunologically active biomolecules like e.g. antibodies as well as immunosuppressively-active chemical compounds. As a non-limiting example, anti-$\mu$M antibodies as described by R.S. Fujinami et al. in *Journal of Virology,* 69, 1995, pp. 5152-5155, the disclosure of which is hereby incorporated by reference, are preferred B-cell depletants according to the present invention. A further example for a B-cell depletant according to the present invention is the LR1 antibody as further described hereinafter. A further example for a B-cell depletant according to the present invention is B220 antibody as further described hereinafter. Also antibodies to malignant B-lymphocytes, useful for the treatment of B-lymphocyte lymphoma, are often cross-reactive with normal B-cells and also can be used for the purposes of the present invention. Examples of such antibodies exist in the literature. E.g. Epstein et al. describe the preparation of two such antibodies, termed Lym-1 and Lym-2, in *Two new Monoclonal Antibodies Lym-1 and Lym-2, Reactive with Human B-Lymphocytes and Derived Tumors, with Immunodiagnostic and Immnuotherapeutic Potential, Cancer Research, 47, 830-840 (1987).* Since it is possible that in some, if not in many cases, the B-cell population may not all share identical surface markers, it may be necessary to utilize more than one antibody to effectively achieve the desired depletion of B-cells. The present invention envisions the utilization of as many antibodies as necessary to accomplish this goal.

Further, the present invention envisions the use of unmodified ('naked') antibodies as well as of antibodies conjugated with a suitable cytotoxic agent, toxin or radionuclide. Appropriate radioisotopes include $^{131}$I, $^{90}$Y, $^{67}$Cu. Procedures for the preparation of iodinated antibodies are well-known in the art and such preparations can be carried out easily in hospital radiopharmacies.

The antibody also can be conjugated, by procedures described in the art with known cytotoxic drugs such as methotrexate, aminopterin, mitoxantrone, vincristine, vinblastine, doxorubicin and others, or with plant toxins such as abrin, or ricin or the like or their ribosome-inactivating sub-units, or any other agents known to have cytotoxic properties.

In addition, the present invention contemplates the use of genetically, enzymatically, or chemically altered antibodies which recognize B-cells, whereby the constant regions have been altered or replaced with domains which fix complement proteins or elicit target cell destruction by virtue of antibody-dependent cellular cytotoxicity (ADCC), thus activating the patient's own immune system.

Further non-limiting examples of B-cell depletants contemplated by the present invention are chemical compounds like ciamexone (US Patent 5 055 290) and imexon (US Patent 5 369 119),

The therapeutic compositions (i.e. the medicaments) of the present invention can be administered parenterally by injection, rapid infusion, nasopharyngeal absorption (intranasopharangally), dermoabsorption, orally, intraocularly, or intracerebroventricularly (i.c.v.). The compositions my alternatively be administered intramuscularly, or intravenously. Compositions for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Carriers or occlusive dressings can be used to increase bioavailability. Liquid dosage forms for oral administration may generally comprise a liposome solution containing the liquid dosage form. Suitable forms for suspending liposomes include emulsions, supensions, solutions, syrups, and elixirs containing inert diluents commonly used in the art, such as purified water. Besides the inert diluents, such compositons can also include adjuvants, wetting agents, emulsifying and suspending agents, or sweetening, flavoring or perfuming agents.

According to the present invention, an "effective amount" of the medicament is one which is sufficient to achieve the desired biological effect. Generally, the dosage needed to provide an effective amount ot the medicament will vary depending upon such factors as the human's or animal's age, condition, sex, and extent of disease, if any, and other variables which can be adjusted by one of ordinary skill in the art.

There is a diagnostic method, which is however not within the scope of the present invention, allowing the determination of the presence or absence of infective B-cells in humans or animals or in body fluid or tissue derived products isolated therefrom. Such assay method comprises the steps of extracting B-cells from body fluids or from tissue or from products derived therefrom and inoculating said B-cells into the cerebrum of a test animal, development of transmissible spongiform encephalopathy in said test animal indicating presence of said infective B-cells. As to the extraction step,

it will be apparent to the man skilled in the art, that such extraction involves use of a reactive physical entity specifically recognizing B-cells, preferably B-cell specific antibodies, as the ones described hereinbelow, Thus, the extraction will preferably be analogous to the separation methods adopted for the manufacture of non-infective body fluid or tissue derived products which are detailed later. In a preferred but not limiting embodiment of the present invention, the inventors have used anti-mouse-B220 antibodies conjugated with super-paramagnetic microbeads (Milteny Biotec GmbH, Germany) for the purification of B-cells.

Suitable test animals for carrying out the method are e.g. *tga20* indicator mice and as they were used by Brandner et al. in 'Normal host prion protein necessary for scrapie-induced neurotoxicity', *Nature. 379, (1996).* As reported by Brandner, infectivity of a given inoculum determines the incubation time elapsed before the appearence of clinical symptoms displayed by the test animals. (see Table 5).

A further object of the invention is the provision of a non-infective human TSE-free body fluid product. Thus, according to the invention, a non-infective body fluid product is a body fluid product which is substantially free of B-cells. A preferred body fluid product according to the invention is a blood product.

A further aspect of the invention is the provision of a non-infective human TSE-free tissue derived product. Thus, according to the invention, a non-infective tissue derived product is a tissue derived product which is substantially free of B-cells. A preferred tissue derived product according to the invention is a product derived from the lymphoreticular system. A still preferred tissue derived product according to the invention is a spleen derived product.

A further aspect of the invention is a method of manufacture of a non-infective human TSE-free body fluid product. Thus, according to the invention, non-infective body fluid products are obtained by specifically depleting in vitro only B-cells from body fluids or from known body fluid products. Though any suitable method known to the man skilled in the art could be used for the specific depletion of B-cells from body fluids, specific depletionby means of B-cell specific immunoreactants like e.g. B-cell specific antibodies is preferred. Suitable but not limiting examples of such B-cell specific antibodies are commercially available B220 or LR1 antibodies or anti- mu M antibodies, vide supra. The term "specific depletion by means of B-cell specific antibodies" encompasses any depletion method which comprises the use of depletion reagents comprising B-cell specific antibodies for the recognition of B-cells in body fluid products. Depletion reagents comprising B-cell specific antibodies are B-cell specific antibodies which are conjugated to a solid phase or which are capable of interacting with a solid phase via chemical or physical means either by themselves or by virtue of suitable derivatization in such a manner that they get either directly or indirectly immobilized on said solid phase so as to enable separation from the reaction mixture.

A further aspect of the invention is a method of manufacture of such a non-infective human TSE-free tissue derived product. Thus, according to the invention, non-infective human TSE-free tissue derived products are obtained by specifically depleting in vitro only B-cells from tissue derived products. Though any suitable method known to the man skilled in the art could be used for the specific depletion of B-cells from tissue derived products, specific depletion by means of B-cell specific immunoreactants like e.g. B-cell specific antibodies is preferred. Suitable but not limiting examples of such B-cell specific antibodies are commercially available B220 or LR1 antibodies or anti- mu M antibodies. The term "specific depletion by means of B-cell specific antibodies" encompasses any depletion method which comprises the use of depletion reagents comprising B-cell specific antibodies for the recognition of B-cells in tissue derived products. Depletant reagents comprising B-cell specific antibodies are B-cell specific antibodies which are conjugated to a solid phase or which are capable of interacting with a solid phase via chemical or physical means either by themselves or by virtue of suitable derivatization in such a manner that they get either directly or indirectly immobilized on said solid phase so as to enable separation from the reaction mixture.

Still further, according to the invention, human TSE-free non-infective body fluid products and/or tissue derived products are isolated from isolated from humans that are only B-cells deficient. Any method known to the man skilled in the art can be used for the depletion of B-cells in organisms. For example, organisms can be treated with anti- mu M antibodies as described by R.S. Fujinami et al. vide supra, so as to become sources of B-cell depleted peripheral blood. A further method for the depletion of B-cells in organisms may be selective knock out of B-cell related genes. A suitable but not-limiting example of an organism obtained by knocking out B-cell related genes is the mu MT mouse described by Kitamira et al. 'A B-cell deficient mouse by targeted disruption of the membrane exon of the immunoglobulin mu-chain gene' *Nature* 350, 423-426 (1991).

Immunoassays: the chapter has an illustrative purpose. Immunoassays are not within the scope of the present invention.

B-cells, shown above to be capable of transmitting spongiform encephalopathy are important for the generation of specific immunological reagents, antigens and antibodies which can be utilized in a variety of assays, many of which are described herein, for the detection of transmissible spongiform encephalopathy (TSE). They can be used as immunogens to produce antibodies. These antibodies can be, for example, polyclonal or monoclonal antibodies, chimeric, single chain and humanized antibodies, as well as Fab fragments, or the product of an Fab expression library. Various procedures known in the art may be used for the production of such antibodies and fragments.

For example, antibodies generated against a preparation of infective B-cells can be obtained by direct injection of the infective 3-cells into an animal. A mouse, rabbit or goat is preferred. The antibody so obtained then will bind the infective B-cells. Such antibodies then can be used to isolate the infective B-cells from test samples such as tissue suspected of containing infectious material. For preparation of monoclonal antibodies, any technique which provides antibodies produced by continuous cell line cultures can be used. Examples include the hybridoma technique as described by Kohler and Milstein, Nature 256:495-497 (1975), the trioma technique, the human B-cell hybridoma technique as described by Kozbor et a1, Immun. Today 4:72 (1983) and the EEV-hybridorna technique to produce human monoclonal antibodies as described by Cole et al., in Monoclonal Antibodies and Cancer Therapy, Alan R. Liss, Inc, New York, NY, pp. 77-96 (1985). Techniques described for the production of single chain antibodies can be adapted to produce single chain antibodies to immunogenic polypeptide products of this invention. See, for example, U.S. Patent No. 4,946,778.

Various assay formats may utilize the antibodies used in the present invention, including "sandwich" immunoassays and probe assays. For example, the antibodies of the present invention, or fragments thereof, can be employed in various assay systems to determine the presence, if any, of infective B-cells in a test sample. For example, in a first assay format, a polyclonal or monoclonal antibody or fragment thereof, or a combination of these antibodies, which has been coated on a solid phase, is contacted with a test sample, to form a first mixture. This first mixture is incubated for a time and under conditions sufficient to form antigen/antibody complexes. Then, an indicator reagent comprising a monoclonal or a polyclonal antibody or a fragment thereof, or a combination of these antibodies, to which a signal generating compound has been attached, is contacted with the antigen/antibody complexes to form a second mixture. This second mixture then is incubated for a time and under conditions sufficient to form antibody/antigen/antibody complexes. The presence of infective B-cells in the test sample and captured on the solid phase, if any, is determined by detecting the measurable signal generated by the signal generating compound. The amount of infective B-cell antigen present in the test sample is proportional to the signal generated.

In an alternative assay format, a mixture is formed by contacting: (1) a polyclonal antibody, monoclonal antibody, or fragment thereof, which specifically binds to infective B-cells , or a combination of such antibodies bound to a solid support; (2) the test sample; and (3) an indicator reagent comprising a monoclonal antibody, polyclonal antibody, or fragment thereof, which specifically binds to a different infective B-cell antigens (or a combination of these antibodies) to which a signal generating compound is attached. This mixture is incubated for a time and under conditions sufficient to form antibody/antigen/antibody complexes. The presence, if any, of infective B-cell antigen present in the test sample and captured on the solid phase is determined by detecting the measurable signal generated by the signal generating compound. The amount of infective B-cell antigen present in the test sample is proportional to the signal generated.

In another assay format, one or a combination of at least two monoclonal antibodies used in the invention can be employed as a competitive probe for the detection of antibodies to infective B-cell antigen. For example, infective B-cells can be gently lysed and coated on a solid phase. A test sample suspected of containing antibody to infective B-cell antigen then is incubated with an indicator reagent comprising a signal generating compound and at least one monoclonal antibody of the invention for a time and under conditions sufficient to form antigen/antibody complexes of either the test sample and indicator reagent bound to the solid phase or the indicator reagent bound to the solid phase. The reduction in binding of the monoclonal antibody to the solid phase can be quantitatively measured.

In yet another detection method, each of the monoclonal or polyclonal antibodies used in the present invention can be employed in the detection of infective B-cell antigens in tissue sections, as well as in cells, by immunohistochemical analysis. Cytochemical analysis wherein these antibodies are labeled directly (with, for example, fluorescein, colloidal gold, horseradish peroxidase, alkaline phosphatase, etc.) or are labeled by using secondary labeled anti-species antibodies (with various labels as exemplified herein) to track the histopathology of disease can also be performed.

In addition, these monoclonal antibodies can be bound to matrices similar to CNBr-activated Sepharose and used for the affinity purification of specific infective B-cells or infective B-cell antigens from cell cultures or biological tissues such as to purify recombinant and native infective B-cell proteins or to prepare biological tissue or fluid devoid of infective B-cells.

The monoclonal antibodies, according to the invention also can be used for the generation of chimeric antibodies for therapeutic use, or other similar applications.

The monoclonal antibodies or fragments thereof can be provided individually to detect infective B-cells. Combinations of the monoclonal antibodies (and fragments thereof) provided herein also may be used together as components in a mixture or 'cocktail' of at least one infective B-cell antibody of the invention, along with antibodies which specifically bind to other infective B-cell regions, each antibody having different binding specificities. Thus, this cocktail can include the monoclonal antibodies of the invention which are directed to infective B-cell polypeptides and other monoclonal antibodies specific to other antigenic determinants of infective B-cells.

The polyclonal antibody or fragment thereof which can be used in the assay formats should specifically bind to an infective B-cell polypeptide or other infective B-cell polypeptides additionally used in the assay. The polyclonal antibody used preferably is of mammalian origin such as, human, goat, rabbit or sheep polyclonal antibody which binds infective B-cells . Most preferably, the polyclonal antibody is of rabbit origin. The polyclonal antibodies used in the assays can be used either alone or as a cocktail of polyclonal antibodies. Since the cocktails used in the assay formats are comprised of either monoclonal antibodies or polyclonal antibodies having different binding specificity to infective B-cells, they are useful for the preventing or treating transmissible spongiform encephalopathy.

Infective B-cells or specific antigens thereof may be detectable in assays by use of a recombinant antigen as well as by use of a synthetic peptide or purified peptide, which peptide comprises an amino acid sequence of infective B-cells. It also is within the scope of the present invention that different synthetic, recombinant or purified peptides, identifying different epitopes of infective B-cells, can be used in combination in an assay for preventing or treating transmissible spongiform encephalopathy. All of these peptides can be coated onto one solid phase; or each separate peptide may be coated onto separate solid phases, such as microparticles, and then combined to form a mixture of peptides which can be later used in assays. Furthermore, it is contemplated that multiple peptides which define epitopes from different antigens may be used for the prevention or treatment of transmissible spongiform encephalopathy. Peptides coated on solid phases or labeled with detectable labels are then allowed to compete with those present in a patient sample (if any) for a limited amount of antibody. A reduction in binding of the synthetic, recombinant, or purified peptides to the antibody (or antibodies) is an indication of the presence of infective B-cells antigen in the patient sample. The presence of infective B-cells antigen indicates the presence of transmissible spongiform encephalopathy in the patient. Variations of assay formats are known to those of ordinary skill in the art and many are discussed herein below.

In another assay format, the presence of anti-infective B-cell antibody and/or infective B-cell antigen can be detected in a simultaneous assay, as follows. A test sample is simultaneously contacted with a capture reagent of a first analyte, wherein said capture reagent comprises a first binding member specific for a first analyte attached to a solid phase and a capture reagent for a second analyte attached to a second solid phase, to thereby form a mixture. This mixture is incubated for a time and under conditions sufficient to form capture reagent/first analyte and capture reagent/second analyte complexes. These so-formed complexes then are contacted with an indicator reagent comprising a member of a binding pair specific for the first analyte labeled with a signal generating compound and an indicator reagent comprising a member of a binding pair specific for the second analyte labeled with a signal generating compound to form a second mixture This second mixture is incubated for a time and under conditions sufficient to form capture reagent/first analyte/indicator reagent complexes and capture reagent/second analyte/indicator reagent complexes. The presence of one or more analytes is determined by detecting a signal generated in connection with the complexes formed on either or both solid phases as an indication of the presence of one or more analytes in the test sample. In this assay format, recombinant antigens derived from the expression systems disclosed herein may be utilized, as well as monoclonal antibodies produced from the proteins derived from the expression systems as disclosed herein. For example, in this assay system, infective B-cell antigen can be the first analyte. Such assay systems are described in greater detail in EP Publication No. 0473065.

In yet other assay formats, the polypeptides disclosed herein may be utilized to detect the presence of antibody against infective B-cell antigen in test samples. For example, a test sample is incubated with a solid phase to which at least one polypeptide such as a recombinant protein or synthetic peptide has been attached. These are reacted for a time and under conditions sufficient to form antigen/antibody complexes. Following incubation, the antigen/antibody complex is detected. Indicator reagents may be used to facilitate detection, depending upon the assay system chosen. In another assay format, a test sample is contacted with a solid phase to which a recombinant protein produced as described herein is attached, and also is contacted with a monoclonal or polyclonal antibody specific for the protein, which preferably has been labeled with an indicator reagent. After incubation for a time and under conditions sufficient for antibody/antigen complexes to form, the solid phase is separated from the free phase, and the label is detected in either the solid or free phase as an indication of the presence of antibody against infective B-cell antigen. Other assay formats utilizing the recombinant antigens disclosed herein are discosed. These include contacting a test sample with a solid phase to which at least one antigen from a first source has been attached, incubating the solid phase and test sample for a time and under conditions sufficient to form antigen/antibody complexes, and then contacting the solid phase with a labeled antigen, which antigen is derived from a second source different from the first source. For example, a recombinant protein derived from a first source such as E. coli is used as a capture antigen on a solid phase, a test sample is added to the so-prepared solid phase, and following standard incubation and washing steps as deemed or required, a recombinant protein derived from a different source (i.e., non-E. coli) is utilized as a part of an indicator reagent which subsequently is detected. Likewise, combinations of a recombinant antigen on a solid phase and synthetic peptide in the indicator phase also are possible. Any assay format which utilizes an antigen specific for infective B-cells produced or derived from a first source as the capture antigen and an antigen specific for infective B-cells from a different second source is contemplated.

Other assays which utilize various other solid phases also are disclosed for example, ion capture procedures for immobilizing an immobilizable reaction complex with a negatively charged polymer (described in EP publication 0326100 and EP publication No. 0406473), can be employed to effect a fast solution-phase immunochemical reaction. An immobilizable immune complex is separated from the rest of the reaction mixture by ionic interactions between the negatively charged poly-anion/immune complex and the previously treated, positively charged porous matrix and detected by using various signal generating systems previously described, including those described in chemiluminescent signal measurements as described in EPO Publication No. O 273,115.

Also, these methods can be adapted for use in systems which utilize microparticle technology including automated and semi-automated systems wherein the solid phase comprises a microparticle (magnetic or nonmagnetic). Such systems include those described in, for example, published EPO applications Nos. EP 0 425 633 and EP 0 424 634, respectively.

The use of scanning probe microscopy (SPM) for immunoassays also is a technology to which the monoclonal antibodies of the present invention are easily adaptable. In scanning probe microscopy, particularly in atomic force microscopy, the capture phase, for example, at least one of the monoclonal antibodies used in the invention, is adhered to a solid phase and a scanning probe microscope is utilized to detect antigen/antibody complexes which may be present on the surface of the solid phase. The use of scanning tunneling microscopy eliminates the need for labels which normally must be utilized in many immunoassay systems to detect antigen/antibody complexes. The use of SPM to monitor specific binding reactions can occur in many ways. In one method, one member of a specific binding partner (analyte specific substance which is the monoclonal antibody of the invention) is attached to a surface suitable for scanning. The attachment of the analyte specific substance may be by adsorption to a test piece which comprises a solid phase of a plastic or metal surface, following methods known to those of ordinary skill in the art. Or, covalent attachment of a specific binding partner (analyte specific substance) to a test piece which test piece comprises a solid phase of derivatized plastic, metal, silicon, or glass may be utilized. Covalent attachment methods are known to those skilled in the art and include a variety of means to irreversibly link specific binding partners to the test piece. If the test piece is silicon or glass, the surface must be activated prior to attaching the specific binding partner. Also, polyelectrolyte interactions may be used to immobilize a specific binding partner on a surface of a test piece by using techniques and chemistries. The preferred method of attachment is by covalent means. Following attachment of a specific binding member, the surface may be further treated with materials such as serum, proteins, or other blocking agents to minimize non-specific binding. The surface also may be scanned either at the site of manufacture or point of use to verify its suitability for assay purposes. The scanning process is not anticipated to alter the specific binding properties of the test piece.

The reagents such as antibodies, proteins and peptides used in the present invention can be utilized in non-solid phase assay systems. These assay systems are known to those skilled in the art.

The reagent employed for the assay can be provided in the form of a test kit with one or more containers such as vials or bottles, with each container containing a separate reagent such as a probe, primer, monoclonal antibody or a cocktail of monoclonal antibodies, or a polypeptide (e.g. recombinantly, synthetically produced or purified) employed in the assay, Other components such as buffers, controls and the like, known to those of ordinary skill in art, may be included in such test kits. Test kits which have means for collecting test samples comprising accessible body fluids, e.g., blood, cerebral spinal fluid, urine, saliva and stool can be used. Such tools useful for collection ('collection materials') include lancets and absorbent paper or cloth for collecting and stabilizing blood; swabs for collecting and stabilizing saliva; cups for collecting and stabilizing urine or stool samples. Collection materials, papers, cloths, swabs, cups and the like, may optionally be treated to avoid denaturation or irreversible adsorption of the sample. The collection materials also may be treated with or contain preservatives, stabilizers or antimicrobial agents to help maintain the integrity of the specimens. Test kits designed for the collection, stabilization and preservation of test specimens obtained by surgery or needle biopsy are also useful. All kits may be configured in two components which can be provided separately; one component for collection and transport of the specimen and the other component for the analysis of the specimen. The collection component, for example, can be provided to the open market user while the components for analysis can be provided to others such as laboratory personnel for determination of the presence, absence or amount of analyte. Further, kits for the collection, stabilization and preservation of test specimens may be configured for use by untrained personnel and may be available in the open market for use at home with subsequent transportation to a laboratory for analysis of the test sample.

The present invention will now be described by way of examples, which are meant to illustrate, but not to limit, the scope of the present invention.

## EXAMPLES

### Example 1

*Generation of t11 $\mu MT$ mice.*

The V-gene segment of the immunoglobulin heavy chain of the immunoglobulin heavy chain of the B-cell hybridoma VI41 (ref. 27) secreting a VSV-neutralizing antibody was cloned into an expression vector encoding the mouse μ-chain of allotype a. Transgenic mice were generated and backcrossed to μMT mice. *t11 $\mu MT$* mice exclusively expressed the transgenic μ-chain of the allotype a; endogenous IgM of the allogtye b and immunoglobulins of other subclasses were not detected in their serum (not shown).

### Example 2

#### 1. Scrapie inoculation

Mice were inoculated with a 1% homogenate of heat- and sarcosyl-treated brain prepared from mice infected with the Rocky Mountain laboratory (RML) scrapie strain. Thirty microliter were used for intra-cranial (i.c.) injection, whereas 100μl were administered by intra-peritoneal (i.p.) route. Mice were monitored every second day, and scrapie was diagnosed according to standard clinical criteria.

#### 2. Western-blot analysis

Ten percent brain homogenates were prepared as described and, where indicated, digested with 20μg/ml of proteinase K for 30 minutes at 37° C. Eighty μg of total protein were then electrophoresed through 12% SDS-polyacrylamide gel, transferred to nitrocellulose membranes, probed with monoclonal antibody 6H4 (Prionics AG, Zurich) or polyclonal antiserum IB3 (reference 26) against mouse PrP, and developed by enhanced chemiluminescence.

3. Detection of PrP antibodies

Brain Lysates from wild-type and *Prnp$^{0/0}$* mice, as well as recombinant *E. coli* PrP, were electrophoresed through a 12,5% SDS-polyacrylamide gel and transferred to nitrocellulose membranes. Membranes were then incubated with serum from infected, terminally scrapie-sick mice (1:100 diluted). Visualization was achieved by enhanced chemiluminescence as previously described for the Western-blot.

4. Immunohistochemical studies

Brain tissues from each mouse was fixed, inactivated for 1 hour with 98% formic acid, embedded in paraffin and subjected to conventional staining and to immuno-staining for glial fibrillary acidic protein according to standard procedure. Glioais (a nonspecific but early indicator of brain damage) was detected by the presence of large immunostained reactive astrocytes. In terminally scrapie-sick mice, wide spread vacuolation was consistently seen throughout the central vervous system.

5. Infectivity bioassays

Brain and spleen homogenate (w/v, 10% in 0,32 M sucrose) were prepared from infected animals as described, and 30μl (diluted 1:10 in phosphate buffered saline containing 1% BSA) wee administered i.c. to groups of at least 4 *tga$^{20}$* mice for each sample. The incubation time until development of terminal scrapie sickness was determined and infectivity titer were calculated using the realtionship $y = 14.37 - 0.11 x$ where $y$ is the ID$_{50}$ and $x$ is the incubation time (in days) to terminal disease.

6. Preparation of solenocytes

Spleens were recovered from mice at 34 days following i.p. inoculation with the RML strain of prions. Splenocyte suspensions were prepared by forcing spleens through a fine mesh screen into 25ml of magnetic activated cell separation (MACS) buffer. The MACS buffer is composed of phosphate buffered saline containing 1% BSA, 5mM EDTA and 0,1% sodium azide. Following a 15 minute incubation on ice to allow the cell clumps to settle the cell suspension was removed for further evaluation.

7. Antibodies

Antibodies conjugated to super-paramagnetic microbeads which specifically recognized B and T cells (anti-mouse-B220, anti-Thy 1,2, anti-IgM, and anti-CD3) were obtained from Milteny Biotech GmbH. All magnetic separation columns (A2 & CS Column) were also obtained from Milteny Biotech GmbH. Rabbit complement was obtained from Cedarlane, Ontario (Low-tox-M rabbit complement). Additional antibodies (LR1, mouse anti-mouse thy 1.2) were obtained from Serotec.

8. B and T cell purification by magnetic bead separation

Five ml of a Splenocyte suspension was centrifuged at 100 rpm for 10 minutes and the cell pellet was recovered in = 0,6ml of MACS buffer. The cells were then incubated with 75μl of B-200 or Lthy 1,2 conjugated super-paramagnetic micrbeads as per manufacturer instruction (Milteny Biotech GmbH) for 15 minutes at 4° C. Following the incubation, the total volume was adjusted to 2ml with MACS buffer and loaded onto a prefilled and washed A2 column (magnetic separation column). Cells not associated with the magnetic microbeads were eluted with 5ml of MACS buffer. The column was then removed from the magnetic field and back flushed to remove the extracted cells. The separation process is then repeated and the final B or T enriched cell population is eluted with 11ml of MACS buffer after the separation column was removed from the magnetic field.

9. Complement lysis

To ruther improve the purity of the B and T cell population abtained by magnetic separation, complement lysis of the T or B cell enriched population was performed. Cells were pelleted and resuspended in cytotoxicity medium (CM, RPMI-1640 media containing 25mM HEPES and 0,3% BSA) to a concentration of 1-3x10$^7$ cells/ml. For B cell depletion, a B cell specific antibody, e.g., LR1 was used. Whereas for TL cell depletion, a T cell specific antibody, e.g., Thy 1,2 was used. Optimal effective antibody concentration would need to be individually determined for the specific antibody sources. Incubation with the antibodies is performed at 4° C for 60 minutes after which the cells were resuspended in LCM containing 20% Low-tox-M rabbit complement and incubated at 37° C for 60 minutes to allow for cell lysis. Viable cells were then separated from the dead cells and debris by centrifugation over lympholyte-M (Cedarlane, Ontario) or other cell separation medium as according to the manufacturer instruction.

10. Cell preparation for Flow Cytometry analysis

Single Singel cell suspension for flow cytometry analysis were prepared in FACS buffer consists of phosphate buffered saline containing 2% FCS, 20mM EDTA and 1% sodium azide. When peripheral blood samples were used, the lymphocyte population was enriched by lysis removal of the red blood cells from heparinized blood. The cell staining process consists of incubating cell population with saturating concentration of fluorescein (FITC)-conjugated antibodies for 30 minutes at 4° C. The cells were then washed with FACS buffer to remove the unbounded material and subject tc flow analysis. When the indirect staining method was used, the cell populations were first incubated with the primary antibody for 30 minutes at 4° C, washed with FACS buffer and followed with an additional 30 minutes of incubation at 4° C with a secondary FITC-conjugated antibody. After removal of the unbounded FITC-conjugated secondary antibodies, the cell populations were then ready for flow analysis.

Discussion of the Results of example 2

1. Determination of scrapie infectivity

Infectivity of brain material from scrapie infected mice was demonstrated by i.c. infection of *tga*20 indicator mice. Infectivity was determined by injecting 30µl samples i.c. into tga20 mice and determining time to disease manifestation by standard histochemical procedure. Table 5 illustrates a typical outcome of such analysis. This analysis gives the success rate of disease transmission and the duration/incubation time for the expression of the disease symptoms. Hence the assay reveal the susceptibility of the host strain to the disease and, thus allow for the determination of the critical cell types necessary for disease transmission.

TABLE 5

| TABLE 5 Determination of scraple infectivity | | | |
|---|---|---|---|
| Source of infectivity | Days after Inoculation | Transmission* | Incubation time of recipient (days) |
| (a) Standard prion inoculum† | | | |
| RML, $10^{-1}$ | - | 4/4 | 59, 60, 63, 68 |
| RML, $10^{-3}$ | - | 2/2 | 66, 67 |
| RML, $10^{-5}$ | - | 4/4 | 84, 85, 85, 96 |
| RML, $10^{-7}$ | - | 1/3 | 109, >217, >217, >217 |
| RML, $10^{-9}$ | - | 0/4 | >217,>217 >217, > 217, > 217, |
| RML, $10^{-11}$ | - | 0/3 | > 217 >217, >217, >217 |

2. Evaluation of the potential target cells for scrapie transmission by genetic methodology

The effect of immune defects on the pathogenesis of scrapie was studied in mice deficient in T cell, B cell or with combined T/B cell defects. A number of different mouse genotypes that are suitable have been generated and the selection of the type to be used will be apparent to a person skilled in the art. The success of infection is determined by examination of the disease symptoms, pathology and by infectivity bioassay. Table 1 illustrate a typical outcome of such analysis. This analysis gives the incubation time from infection to symptom presentation, the presence or absence of symptoms and pathological features. Further the infectivity bioassay provide information regarding the latency of the infective agents in the brain and splenic tissues of the primary infected host. By correlating the disease expression and genotype of infected animals, table 1 illustrates that if the infective agent is introduced by the i.c. route all genotypes express the disease regardless of their B cell or T cell defects. Alternatively, by examining the (secondary) infective capability of brain and splenic tissues from the primary infected hosts, the potential target cell lineage of scrapie transmission can be examined. Thus table 2 further illustrates that following i.c. inoculation, only those genotypes with intact B cell functions are capable of demonstrating secondary infectivity in the spleen tissues.

By taking a more peripheral route of primary infection, i.e., i.p. inoculation, the propagation of the disease can be further delineated. This is further illustrated in table 1. The analysis demonstrates that by selecting animals with specific lymphocyte defects, the critical lymphoid cell types for scrapie disease transmission can be specifically identified. These results suggest that B cells may "transport" prions from lymphoid organs to nervous tissues. (The mode of transport is not limited to direct cell associated transport but may also be complexes with various cellular products. The components is not limited to but may include antibodies, PrP$^c$, PrP$^{sc}$ and other similar cellular products).

3. Evaluation of the role of lymphoid cells in prion disease transmission

Cellular components of the peripheral lymphoid tissues, e.g., spleen, lymph nodes can be readily obtained from animals. Such conditions are described by public literature.

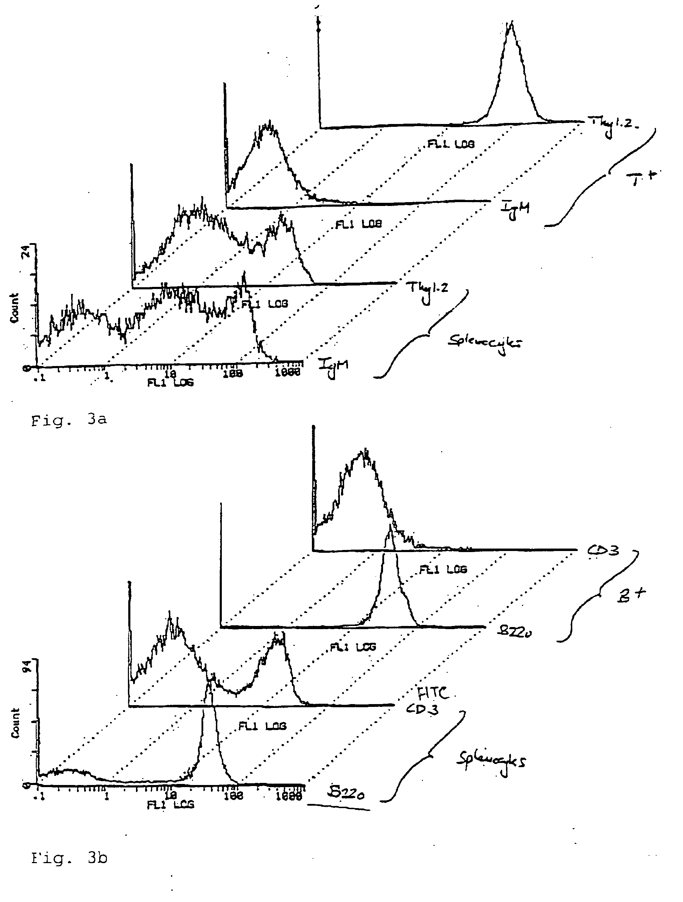

The cellular components obtained can be further separated by specific antibody to differential surface markers for the various lymphoid cell types which gas been conjugated to magnetic microbeads. By additional deletion.of undesirable cell types by cytotoxic depletion using complement, highly purified cell isolates can be obtained. The procedure is constructed to isolate highly enriched T-cell and B-cell populations. The isolated cell populations are suspended in culture medium, e.g., RPMI-1640 and can be supplemented with serum and with additives like glutamic acid, growth factors, cytokines or other modulators of cell physiology prior to evaluation of infectivity capacity. Such highly enriched lymphocytes can be further characterized by Flow cytometry evaluation of the membrane surface components, e.g., CD-4, CD-8, and/or Ig expression and is obvious to a person skilled in the art. Figure 3a and 3b illustrate a typical Flow analysis of such enriched population. The cellular purity is demonstrated by the expression of T cell or B cell specific sufrace markers. Other non cell lineage associated components can also be documented by similar means, e.g., cell surface expression of PrP$^c$ and PrP$^{sc}$. Further, molecular biology techniques as described by public literature can also be employed to document non-membrane associated specific intracellular components, e.g., DNA, RNA, mRNA whose presence is indicative of ist cellular presence.

Such cellular lymphoid components can be obtained from infective and non-infective hosts and characterized for ist lineage and intracellular capacities. Subsequently, their infective capacity can be examined by inoculation via the i.c. or i.p. route. By this assay it is possible to determine the cell lineage most responsible for prion disease transmission. Further, by measurement of various intracellular components and correlation with the cellular lineage, the assay is indicative of the interactions between the prions and the tentative target cells.

TABLE 6

| Table 6 Infectivity In cell fractions of Tg94 and wt spleen | | | | | | | |
|---|---|---|---|---|---|---|---|
| | | Tg94 (1. Experiment) | | Tg94 (2. Experiment) | | wt | |
| Fraction | Cells | Inc. time | n/no | Inc. time | n/no | Inc. time | n/no |
| splenocytes | $10^6$ | 76 ± 4 | (4/4) | nd | | nd | |
| | $10^5$ | 84.5 ±1 | (2/2) | 89 ± 1 | (2/2) | 83.5 ±3 | (2/2) |
| | $10^4$ | 109±14 | (4/4) | 106.5 ± 18 | (4/4) | 89 ± 5 | (4/4) |
| | $10^3$ | 142 | (1/4) | 116.5±12 | (2/4) | 106 ± 7 | (4/4) |
| | $10^2$ | 141±35 | (2/2) | >200 | (0/4) | >200 | (0/4) |
| B cells | 2x$10^5$ | 76±1 | (4/4) | nd | | nd | |
| | 2x$10^4$ | 87±1 1 | (3/4) | 106±10 | (4/4) | 88 ± 2 | (4/4) |
| | 2x$10^3$ | 116±37 | (3/4) | 106±11 | (2/4) | 102±14 | (4/4) |
| | 2x$10^2$ | 110 | (1/4) | >200 | (0/4) | 91 | (1/4) |
| T cells | $10^5$ | 82 ±6 | (4/4) | nd | | nd | |
| | $10^4$ | 110±10 | (3/4) | 109.5±1 | (2/4) | 94±8 | (4/4) |
| | $10^3$ | 106 | (1/3) | >200 | (0/4) | 108±18 | (2/2) |
| | $10^2$ | 108 | (1/4) | >200 | (0/4) | >200 | (0/4) |
| Non B/T cells | 5x$10^5$ | 124.5±1 | (2/4) | 108 | (1/4) | 112±14 | (3/4) |
| | 5x$10^4$ | | (0/4) | nd | | >200 | (0/4) |

FACS analysis shown in Fig. 2D

Peripheral blood cells were incubated with serum from t11μMT mice, washed, incubated with anti-mouse IgM-FITC conjugate followed by anti-CD3-PE (Pharmingen), and analysed with a Becton-Dickinson FAScan instrument after erythrocyte lysis and fixation. For analysis, cells were gated on CD3-positive T-cells. EL4 cells infected with vesicular stomatitis virus (VSV) were stained with 5μg VSV-specific monoclonal antibody VI24 (ref.27) and with FTC-labelled antibody to mouse IgG2a (Southern Biotechnology), or with serum of t11μMT mice, and with FITC-labelled F(ab')2 antibody to mouse IgM (anti-IgM-FITC, Tago), or with serum of C57BL/6 mice and anti-IgM-FITC. All data acquisition and analysis were performed with CellQuest software (Becton Dickinson) .

**Example 4**

Production of Antibodies Against Infective Lymphocytes

A. Production of Polyclonal Antisera.

Antiserum against infective lymphocytes (B-cells or T-cells) is prepared by injecting appropriate animals with infective lymphocytes identified and isolated as described in example 2.

1. Starting materials

Specifically, purified B-cell peparations and/or T-cell preparations are used. The whole cell preparations of infective lymphocytes can be used directly as immunogen or alternatively infective lymphocytes can be gently lysed with mild detergent treatment for example with 0.05-0.5% Triton X 100 followed by fixation in 0.5-2% paraformaldehyde in 1% PBS for 5-100 minutes at 4-10° C.

2. Animal Immunization. Female white New Zealand rabbits weighing 2 kg or more are used for raising polyclonal antiserum. Generally, one animal is immunized per infective lymphocyte preparation. One week prior to the first immunization, 5 to 10 ml of blood is obtained from the animal to serve as a non-immune prebleed sample.

Infective lymphocytes are used to prepare the primary immunogen by emulsifying 0.5 ml of the infective lymphocyte preparation at a concentration of between $1 \times 10^5$ to $1 \times 10^2$ cells/ml in PBS (pH 7.2) with 0.5 ml of complete Freund's adjuvant (CFA) (Difco, Detroit, MI). The immunogen is injected into several sites of the animal via subcutaneous, intraperitoneal, and/or intramuscular routes of administration. Four weeks following the primary immunization, a booster immunization is administered. The immunogen used for the booster immunization dose is prepared by emulsifying 0.5 ml of the same infective lymphocyte preparation used for the primary immunogen, except that 0.5 ml of incomplete Freund's adjuvant (IFA) (Difco, Detroit, MI) is now used. Again, the booster dose is administered into several sites and can utilize subcutaneous, intraperitoneal and intramuscular types of injections. The animal is bled (5 ml) two weeks after the booster immunization and the serum is tested for immunoreactivity to the infective lymphocyte preparation as described below. The booster and bleed schedule is repeated at 4 week intervals until an adequate titer is obtained. The titer or concentration of antiserum is determined by microtiter EIA as described in Example 17, below. An antibody titer of 1:500 or greater is considered an adequate titer for further use and study.

B. Production of Monoclonal Antibody.

1. Immunization Protocol. Mice are immunized using immunogens prepared as described hereinabove, except that the amount of the immunogen for monoclonal antibody production in mice is one-tenth the amount used to produce polyclonal antisera in rabbits. The primary immunogen consists of 0.1ml of the infective lymphocyte preparation at a concentration of between $1 \times 10^5$ to $1 \times 10^8$ cells/ml in PBS (pH 7.2) in 0.1 ml of CFA emulsion; while the immunogen used for booster immunizations consists of 0.1ml of the infective lymphocyte preparation as above emulsified with 0.1 ml of IFA. Hybridomas for the generation of monoclonal antibodies are prepared and screened using standard techniques. The methods used for monoclonal antibody development follow procedures known in the art such as those detailed in Kohler and Milstein, Nature 256:494 (1975) and reviewed in J.G.R. Hurrel, ed., Monoclonal Hybridoma Antibodies: Techniques and Applications, CRC Press, Inc., Boca Raton, FL (1982). Another method of monoclonal antibody development which is based on the Kohler and Milstein method is that of L.T. Mimms et al., Virology 176:004-619 (1990), which is incorporated herein by reference.

The immunization regimen (per mouse) consists of a primary immunization with additional booster immunizations. Booster immunizations are performed at approximately two weeks and four weeks post primary immunization. A total of 100 μl of immunogen is inoculated intraperitoneally and subcutaneously into each mouse. Individual mice are screened for immune response by microtiter plate enzyme immunoassay (EIA) as described in Example 17 approximately four weeks after the third immunization. Mice are inoculated either intravenously, intrasplenically or intraperitoneally with 0.1ml of the infective lymphocyte preparation at a concentration of between $1 \times 10^5$ to $1 \times 10^8$ cells/ml in PBS (pE 7.2) in 0.1 ml of IFA approximately fifteen weeks after the third immunization..

Three days after this intravenous boost, splenocytes are fused with, for example, Sp2/0-Ag14 myeloma cells (Milstein Laboratories, England) using the polyethylene glycol (PEG) method. The fusions are cultured in Iscove's Modified Dulbecco's Medium (IMDM) containing 10% fetal calf serum (FCS), plus 1% hypoxanthine, aminopterin and thymidine (HAT). Bulk cultures are screened by microtiter plate EIA following the protocol in Example 17. Clones reactive with the infectious lymphocyte preparation used as immunogen and non-reactive with non-infectious lymphocyte preparation (i.e., lymphocytes prepared from non-infected animals not used as the immunogen) are selected for final expansion. Clones thus selected are expanded, aliquoted and frozen in IMDM containing 10% FCS and 10% dimethyl-sulfoxide.

2. Production of ascites Fluid Containing Monoclonal Antibodies. Frozen hybridoma cells prepared as described hereinabove are thawed and placed into expansion culture. Viable hybridoma cells are inoculated intraperitoneally into Pristane treated mice. Ascites fluid is removed from the mice, pooled, filtered through a 0.2 $\mu$ filter and subjected to an immunoglobulin class G (IgG) analysis to determine the volume of the Protein A column required for the purification.

3. Purification of Monoclonal Antibodies From Ascites Fluid. Briefly, filtered and thawed ascites fluid is mixed with an equal volume of Protein A sepharose binding buffer (1.5 M glycine, 3.0 M NaCl, pH 8.9) and refiltered through a 0.2 $\mu$ filter. The volume of the Protein A column is determined by the quantity of IgG present in the ascites fluid. The eluate then is dialyzed against PBS (pH 7.2) overnight at 2-8 $_{i}$ C. The dialyzed monoclonal antibody is sterile filtered and dispensed in aliquots. The immunoreactivity of the purified monoclonal antibody is confirmed by determining its ability to specifically bind to the infectious lymphocyte preparation used as the immunogen by use of the EIA microtiter plate assay procedure of Example 17. The specificity of the purified monoclonal antibody is confirmed by determining its lack of binding to irrelevant non-infectious lymphocytes not used as the immunogen. The purified anti-infectious lymphocyte monoclonal thus prepared and characterized is placed at either 2-8 ; C for short term storage or at -80 ; C for long term storage.

4. Further Characterization of Monoclonal Antibody. The isotype and subtype of the monoclonal antibody produced as described hereinabove can be determined using commercially available kits (available from Amersham. Inc., Arlington Heights, IL). Stability testing also can be performed on the monoclonal antibody by placing an aliquot of the monoclonal antibody in continuous storage at 2-8$_{i}$C and assaying optical density (OD) readings throughout the course of a given period of time.

C. Use of Recombinant Proteins as Immunogens. It is within the scope of the present invention that recombinant proteins made as described herein can be utilized as immunogens in the production of polyclonal and monoclonal antibodies, with corresponding changes in reagents and techniques known to those skilled in the art.

## Example 5

Purification of Serum Antibodies Which Specifically Bind to Infectious Lymphocytes

Immune sera, obtained as described hereinabove in Example 14, is affinity purified using immobilized proteins from the infectious lymphocyte preparation used as the immunogen as described above. An IgG fraction of the antiserum is obtained by passing the diluted, crude antiserum over a Protein A column (Affi-Gel protein A, Bio-Rad, Hercules, CA). Elution with a buffer (Binding Buffer, supplied by the manufacturer) removes substantially all proteins that are not immunoglobulins. Elution with 0.1M buffered glycine (pH 3) gives an immunoglobulin preparation that is substantially free of albumin and other serum proteins.

Immunoaffinity chromatography is performed to obtain a preparation with a higher fraction of specific antigen-binding antibody. The infectious lymphocyte preparation used to raise the antiserum is immobilized on a chromatography resin, and the specific antibodies directed against its epitopes are adsorbed to the resin. After washing away non-binding components, the specific antibodies are eluted with 0.1 M glycine buffer, pH 2.3. Antibody fractions are immediately neutralized with 1.0M Tris buffer (pH 8.0) to preserve immunoreactivity. A resin such as Affi-Gel 10 or Affi-Gel 15 is used (Bio-Rad, Hercules, CA). If coupling through a carboxy is desired, Affi-Gel 102 can be used (Bio-Rad, Hercules, CA). An organomercurial resin such as Affi-Gel 501 can be used (Bio-Rad, Hercules, CA).

Alternatively, spleens can be harvested and used in the production of hybridomas to produce monoclonal antibodies following routine methods known in the art as described hereinabove.

## Example 6

Western Blotting of Tissue Samples

Protein extracts are prepared by homogenizing tissue samples in 0.1M Tris-HCl (pH 7.5), 15% (w/v) glycerol, 0.2mM EDTA, 1.0 mM 1,4-dithiothreitol, 10 $\mu$g/ml leupeptin and 1.0 mM phenylmethylsulfonylfluoride (Kain et al., Biotechniques, 17:982 (1994)). Following homogenization, the homogenates are centrifuged at 4°C for 5 minutes to separate supernate from debris. For protein quantitation, 3-10 $\mu$l of supernate are added to 1.5 ml of bicinchoninic acid reagent (Sigma, St. Louis, MO), and the resulting absorbance at 562 nm is measured.

For SDS-PAGE, samples are adjusted to desired protein concentration with Tricine Buffer (Novex, San Diego, CA), mixed with an equal volume of 2X Tricine sample buffer (Novex, San Diego,CA), and heated for 5 minutes at 100$_{i}$C in a thermal cycler. Samples are then applied to a Novex 10-20% Precast Tricine Gel for electrophoresis. Following electrophoresis, samples are transferred from the gels to nitrocellulose membranes in Novex Tris-Glycine Transfer buffer. Membranes are then probed with specific anti-infective lymphocyte antibodies using the reagents and procedures provided in the Western Lights or Western Lights Plus (Tropix, Bedford, MA) chemiluminesence detection kits. Chemiluminesent bands are visualized by exposing the developed membranes to Hyperfilm ECL (Amersham, Arlington Heights, IL).

Competition experiments are carried out in an analogous manner as above, with the following exception; the primary antibodies (anti-infective lymphocyte polyclonal antisera) are pre-incubated for 30 minutes at room temperature with varying concentrations of non-infective lymphocyte immunogen prior to exposure to the nitrocellulose filter. Development of the Western is performed as above.

After visualization of the bands on film, the bands can also be visualized directly on the membranes by the addition and development of a chromogenic substrate such as 5-bromo-4-chloro-3-indolyl phosphate (BCIP). This chromogenic solution contains 0.016% BCIP in a solution containing 100 mM NaCl, 5 mM $MgCl_2$ and 100 mM Tris-HCl (pH 9.5). The filter is incubated in the solution at room temperature until the bands develop to the desired intensity. Molecular mass determination is made based upon the mobility of pre-stained molecular weight standards (Novex, San Diego, CA) or biotinylated molecular weight standards (Tropix, Bedford, MA).

## Example 7

EIA Microtiter Plate Assay

The immunoreactivity of antiserum preferably obtained from rabbits or mice as described in Example 14 is determined by means of a microtiter plate EIA, as follows. Protein from infectious or non-infectious lymphocyte preparations as described above (EXAMPLE 2) is prepared by homogenization of lymphocytes in an appropriate buffer for example PBS (7.2) or with a mild detergent such as 0.01% Triton X 100. Next, 100 μl of the above protein solution is placed in each well of an Immulon 2® microtiter plate (Dynex Technologies, Chantilly, VA). The plate is incubated overnight at room temperature and then washed four times with deionized water. The wells are blocked by adding 125 μl of a suitable protein blocking agent, such as Superblock® (Pierce Chemical Company, Rockford, IL), in phosphate buffered saline (PBS, pH 7.4) to each well and then immediately discarding the solution. This blocking procedure is performed three times. Antiserum obtained from immunized rabbits or mice prepared as previously described is diluted in a protein blocking agent (e.g., a 3% Superblock® solution) in PBS containing 0.05% Tween-20® (monolaurate polyoxyethylene ether) (Sigma Chemical Company, St. Louis, MO) and 0.05% sodium azide at dilutions of 1:500, 1:2500, 1:12,500, 1:62,500 and 1:312,500 and placed in each well of the coated microtiter plate. The wells then are incubated for three hours at room temperature. Each well is washed four times with deionized water. One hundred μl of alkaline phosphatase-conjugated goat anti-rabbit IgG or goat anti-mouse IgG antiserum (Southern Biotech, Birmingham, AB), diluted 1:2000 in 3% Superblock® solution in phosphate buffered saline containing 0.05% Tween 20® and 0.05% sodium azide, is added to each well. The wells are incubated for two hours at room temperature. Next, each well is washed four times with deionized water. One hundred microliters (100 μl) of paranitrophenyl phosphate substrate (Kirkegaard and Perry Laboratories, Gaithersburg, MD) then is added to each well. The wells are incubated for thirty minutes at room temperature. The absorbance at 405 nm is read of each well. Positive reactions are identified by an increase in absorbance at 405 nm in the test well above that absorbance given by a non-immune serum (negative control). A positive reaction is indicative of the presence of detectable anti-infective lymphocyte antibodies.

In addition to titers, apparent affinities [$K_d$(app)] may also be determined for some of the antisera. EIA microtiter plate assay results can be used to derive the apparent dissociation constants ($K_d$) based on an analog of the Michaelis-Menten equation (V. Van Heyningen, Methods in Enzymology, Vol. 121, p. 472 (1986) and further described in X. Qiu, et al Journal of Immunology, Vol. 156, p. 3350 (1996)):

$$[Ag\text{-}Ab] = [Ag\text{-}Ab]_{max} \quad X \quad \frac{[Ab]}{[Ab] = K_d}$$

Where [Ag-Ab] is the antigen-antibody complex concentration, [Ag-Ab]$_{max}$ is the maximum complex concentration, [Ab] is the antibody concentration, and $K_d$ is the dissociation constant. During the curve fitting, the [Ag-Ab] is replaced with the background subtracted value of the $OD_{405nm}$ at the given concentration of Ab. Both $K_d$ and [$OD_{405nm}$]$_{max}$, which corresponds to the [Ag-Ab]$_{max}$, are treated as fitted parameters. The software program Origin can be used for the curve fitting.

**Example 8**

Coating of Solid Phase Particles

A. Coating of Microparticles with Antibodies Which

Specifically Bind to Infective Lymphocytes. Affinity purified antibodies which specifically bind to infective lymphocytes (see Example 15) are coated onto microparticles of polystyrene, carboxylated polystyrene, polymethylacrylate or similar particles having a radius in the range of about 0.1 to 20 $\mu$m. Microparticles may be either passively or actively coated. One coating method comprises coating EDAC (1-(3-dimethylaminopropyl)-3-ethylcarbodiimide hydrochloride (Aldrich Chemical Co., Milwaukee, WI) activated carboxylated latex microparticles with antibodies which specifically bind to infective lymphocytes , as follows. Briefly, a final 0.375% solid suspension of resin washed carboxylated latex microparticles (available from Bangs Laboratories, Carmel, IN or Serodyn, Indianapolis, IN) are mixed in a solution containing 50 mM MES buffer, pH 4.0 and 150 mg/l of affinity purified anti-infective lymphocyte antibody (see Example 14) for 15 min in an appropriate container. EDAC coupling agent is added to a final concentration of 5.5 $\mu$g/ml to the mixture and mixed for 2.5 h at room temperature.

The microparticles then are washed with 8 volumes of a Tween 20®/sodium phosphate wash buffer (pH 7.2) by tangential flow filtration using a 0.2 $\mu$m Microgon Filtration module. Washed microparticles are stored in an appropriate buffer which usually contains a dilute surfactant and irrelevant protein as a blocking agent, until needed.

B. Coating of 1/4 Inch Beads. Antibodies which specifically bind to infective lymphocyte antigen also may be coated on the surface of 1/4 inch polystyrene beads by routine methods known in the art (Snitman et al, US Patent 5,273,882, incorporated herein by reference) and used in competitive binding or EIA sandwich assays.

Polystyrene beads first are cleaned by ultrasonicating them for about 15 seconds in 10 mM NaHCO$_3$ buffer at pH 8.0. The beads then are washed in deionized water until all fines are removed. Beads then are immersed in an antibody solution in 10 mM carbonate buffer, pH 8 to 9.5. The antibody solution can be as dilute as 1 $\mu$g/ml in the case of high affinity monoclonal antibodies or as concentrated as about 500 $\mu$g/ml for polyclonal antibodies which have not been affinity purified. Beads are coated for at least 12 hours at room temperature, and then they are washed with deionized water. Beads may be air dried or stored wet (in PBS, pH 7.4). They also may be overcoated with protein stabilizers (such as sucrose) or protein blocking agents used as non-specific binding blockers (such as irrelevant proteins, Carnation skim milk, Superblock®, or the like).

Example 9: Illustrative Example.

Microparticle Enzyme Immunoassay (MEIA)

Infective lymphocyte antigens are detected in patient test samples by performing a standard antigen competition EIA or antibody sandwich EIA and utilizing a solid phase such as microparticles (MEIA). The assay can be performed on an automated analyzer such as the IMx® Analyzer (Abbott Laboratories, Abbott Park, IL).

A. Antibody Sandwich BIA. Briefly, samples suspected of containing infective lymphocyte antigen are incubated in the presence of anti-infective lymphocyte antibody-coated microparticles (prepared as described in Example 17) in order to form antigen/antibody complexes. The microparticles then are washed and an indicator reagent comprising an antibody conjugated to a signal generating compound (i.e., enzymes such as alkaline phosphatase or horseradish peroxidase) is added to the antigen/antibody complexes or the microparticles and incubated. The microparticles are washed and the bound antibody/antigen/antibody complexes are detected by adding a substrate (e.g., 4-methyl umbelliferyl phosphate (MUP), or OPD/peroxide, respectively), that reacts with the signal generating compound to generate a measurable signal. An elevated signal in the test sample, compared to the signal generated by a negative control, detects the presence of infective lymphocyte antigen. The presence of infective lymphocyte antigen in the test sample is indicative of a diagnosis of transmissible spongiform encephalopathy (TSE).

B. Competitive Binding Assay. The competitive binding assay uses a protein or proteins from an infective lymphocyte preparation that generates a measurable signal when the labeled protein is contacted with an anti-infective lymphocyte antibody coated microparticle. This assay can be performed on the IMx® Analyzer (available from Abbott Laboratories, Abbott Park, IL). The labeled proteins from an infective lymphocyte preparation are added to the infective lymphocyte antibody-coated microparticles (prepared as described in Example 17) in the presence of a test sample suspected of containing infective lymphocyte antigen, and incubated for a time and under conditions sufficient to form labeled infective lymphocyte protein / bound antibody complexes and/or patient infective lymphocyte antigen / bound antibody complexes. The infective lymphocyte antigen in the test sample competes with the labeled infective lymphocyte proteins for binding sites on the microparticle. Infective lymphocyte antigen in the test sample results in a lowered binding of labeled infective lymphocyte protein and antibody coated microparticles in the assay since antigen in the test sample and the infective lymphocyte protein compete for antibody binding sites. A lowered signal (compared to a control) indicates the presence of infective lymphocyte antigen in the test sample. The presence of infective lymphocyte antigen suggests the diagnosis of TSE.

The infective lymphocyte proteins discussed hereinabove are useful as markers of TSE. Tests based upon the appearance of this marker or markers in a test sample such as blood, serum, plasma, cerebral spinal fluid, and tissues can provide low cost, non-invasive, diagnostic information to aid the physician to make a diagnosis of TSE, to help select a therapy protocol, or to monitor the success of a chosen therapy. This marker or markers may appear in readily accessible body fluids such as blood, urine, CSF, or stool as antigens derived from the diseased tissue which are detectable by immunological methods. This marker may be elevated in a disease state, altered in a disease state, or be a normal protein which appears in an inappropriate body compartment, in an altered state or form indicative of disease.

## References cited in the present Application

1. Hill, A. F. et al. The same prion strain causes vCJD and BSE. *Nature* 389, 448-450 (1997).

2. Bruce, M. E. et al. Transmissions to mice indicate that 'new variant' CID is caused by the BSE agent. *Nature* 389, 498-501 (1997).

3. Kitamoto, T., Muramoto, T., Mohri, S., Dohura, K. & Tateishi, J. Abnormal isoform of prion protein accumulates in follicular dendritic dells in mice with Creutzfeldt-Jakob disease. *J. Virol.* 65, 6292-6295 (1991).

4. Lasmezas, C. I. et al. Immune system-dependent and-independent replication of the scrapie agent: *J. Virol.* 70, 1292-1295 (1996).

5. Shinkai, Y. et al. RAG-2-deficient mice lack mature lymphocytes owing to inability to initiate V(D) J rearrangement. *Cell* 68, 855-867 (1992).

6. Mombaerts, P. et al. RAG-1-deficient mice have no mature B and T lymphocytes. *Cell* 68, 869-877 (1992).

7. Huang, S. et al. Immune response in mice that lack the interferon-y receptor. *Science* 259, 1742-1745 (1993).

8. Müller, U. et al. Functional role of type I and type II interferons in antiviral defense. *Science* 264, 1918-1921 (1994).

9. Rahemtulla, A. et al. Normal development and function of CD8[+] cells but markedly decreased helpercell activity in mice lacking CD4. *Nature* 353, 180-184 (1991).

10. Fung Leung, W. P. et al. CD8 is needed for development of cytotoxic T cells but not helper T cells. *Cell* 65, 143-449 (1991).

11. Ziilstra, M. et al. β 2-Microgiobuin-deficient mice lack CD4[+]8[+] cytolytic T cells. *Nature* 344, 742-746 (1990).

12. Kägi, D. et al. Cytotoxicity mediated by T cells and natural killer cells is greatly impaired in perforindeficient mice, *Nature* 369, 31-37 (1994).

13. Kitamura, D., Roes, J., Kuhn, R. & Rajewsky, K. A B-cell-deficient mouse by targeted disruption of the membrane exon of the immunoglobulin Mu-chain gene. *Nature* 350, 423-426 (1991).

14. Fischer, M. et al. Prion protein (PrP) with amino-proximal deletions rstoring susceptibility of PrP-knockout mice to scrapie. *EMBO J.* 15, 1255-1264 (1996).

15. Büeler, H. R. et al. Normal development and behaviour of mice lacking the neuronal cell-surface PrP protein. *Nature* 356, 577-582 (1992).

16. Büeler, H. R. et al. Mice devoid of PrP are resistant to scrapie. *Cell* 73, 1339-1347 (1993).

17. Fraser, H. et al. Replication of scrapie in spleens of scid mice follows reconstitution with wild-type mouse bone marrow. *J. Gen. Virol.* 77, 1935-1940 (1996).

18. Nonoyama, S., Smith, F. O., Bernstein, I. D. & Ochs, H. D. Strain-dependent leakiness of mice with severe combined immune deficiency. *J. Immunol.* 150, 3817-3824 (1993).

19. Bosma, M. J. & Carroll, A. M. The SCID mouse mutant: definition, characterization, and potential uses. Annu. Rev. Immunol. 9, 323-350 (1991).

20. Eigen, M. Prionics or the kinetic basis of prion diseases. *Biophys. Chem.* 63, A1-18 (1996).

21. Hill, A. F., Zeidler, M., Ironside, J. & Collinge, J. Diagnosis of new variant Creutzfeldt-Jakob disease by tonsil biopsy. Lancet 349, 99 (1997).

22. Rothe, J. et al. Mice lacking the tumour-necrosis factor receptor 1 are resistant to TNF-mediated toxicity but highly susceptible to infection by Listeria monocytogenes. *Nature* 364, 798-802 (1993).

23. Le Hir, M. et al. Differentiation of follicular dendritic cells and full antibody responses require tumor necrosis factor receptor-1 signaling. *J. Exp. Med.* 183, 2367-2372 (1996).

24. Humphrey, J. H., Grennan, D. & Sundaram; V. The origin of follicular dendritic cells in the mouse and the mechanism of trapping of immune complexes on them. *Eur. J. Immunol.* 14, 859-864 (1984).

# EP 0 931 551 B1

25. Blättler, T. et al. Transfer of scrapie infectivity from spleen to brain depends on interposed PrP-expressing tissue. *Nature* 389, 69-73 (1997).

26. Farquhar, C. F., Somerville, R. a. & Ritchie, L. A. Post-mortem immunodiagnosis of scrapie and bovine spongiform encephalopathy. *J. Virol. Meth.* 24, 215-221 (1989).

27. Kalinke, U. et al. The role of somatic mutation in the generation of the protective humoral immune response against vesicular stomatitis virus. *Immunity* 5, 639-652 (1996).

28. Prusiner, S. B. et al. Measurement of the scrapie agent using an incubation time interval assay. *Neurol.* 11, 353-358 (1982).

29. Brandner, S. et al. Normal host prion protein (PrPc) required for scrapie spread within the central nervous system. *Proc. Natl Acad. Sci.* USA 93, 13148-13151 (1996).

## Claims

1. Use of selective B-cell depletants for the manufacture of a medicament for the treatment or prevention of transmissible spongiform encephalopathy in infected humans or animals.

2. Use according to claim 1, **characterized in that** said B-cell depletants comprise immunologically active biomolecules.

3. Use according to claim 2 **characterized in that** said immunologically active biomolecules comprise antibodies.

4. Use according to claim 3, **characterized in that** said antibodies comprise anti-μM antibodies.

5. Use according to claim 3, **characterized in that** said antibodies comprise LR1 antibodies.

6. Use according to claim 3, **characterized in that** said antibodies comprise B220 antibodies.

7. Use according to claim 1, **characterized in that** said B-cell depletants comprise immunosuppressively active chemical compounds.

8. Use according to claim 7, **characterized in that** said immunosuppressively active chemical compounds comprise ciamexone.

9. Use according to claim 7, **characterized in that** said immunosuppressively active chemical compounds comprise imexon.

10. Human TSE-free body fluid or tissue derived product, **characterized in that** said body fluid or tissue derived product has been depleted only of B-cells in vitro by being treated with a medicament comprising a selective B-cell depletant.

11. TSE-free body fluid or tissue derived product according to claim 10, **characterized in that** said B-cell depletant comprises immunologically active biomolecules.

12. TSE-free body fluid or tissue derived product according to claim 11 **characterized in that** said immunologically active biomolecules comprise antibodies.

13. TSE-free body fluid or tissue derived product according to claim 12 **characterized in that** said antibodies comprise anti-μM antibodies.

14. TSE-free body fluid or tissue derived product according to claim 12 **characterized in that** said antibodies comprise LR1 antibodies.

15. TSE-free body fluid or tissue derived product according to claim 12 **characterized in that** said antibodies comprise B220 antibodies.

16. TSE-free body fluid or tissue derived product according to claim 10, **characterized in that** said B-cell depletant comprises immunosuppressively active chemical compounds.

17. TSE-free body fluid or tissue derived product according to claim 16, **characterized in that** said immunosuppressively active chemical compounds comprise ciamexone.

18. TSE-free body fluid or tissue derived product according to claim 16, **characterized in that** said immunosuppressively active chemical compounds comprise imexon.

19. Method for preparing a human TSE-free body fluid or tissue derived product, **characterized in that** said method comprises a step of depleting only B-cells from said body fluid or tissue derived product *in vitro,* wherein the B-cells are depleted by means of a medicament comprising a selective B-cell depletant.

20. Method according to claim 19, **characterized in that** said B-cell depletant comprises immunologically active biomolecules.

21. Method according to claim 20 **characterized in that** said immunologically active biomolecules comprise antibodies.

22. Method according to claim 21, **characterized in that** said antibodies comprise anti-$\mu$M antibodies.

23. Method according to claim 21, **characterized in that** said antibodies comprise **LR1 antibodies.**

24. Method according to claim 21, **characterized in that** said antibodies comprise B220 antibodies.

25. Method according to claim 19, **characterized in that** said B-cell depletant comprises immunosuppressively active chemical compounds.

26. Method according to claim 25, **characterized in that** said immunosuppressively active chemical compounds comprise ciamexone.

27. Method according to claim 25, **characterized in that** said immunosuppressively active chemical compounds comprise imexon.

28. Method for isolating human TSE-free body fluid or tissue derived products, **characterized in that** said body fluid or tissue derived products are isolated from non-living humans that are only B-cell deficient.

29. Human TSE-free body fluid or tissue derived product, **characterized in that** said body fluid or tissue derived product is isolated from humans that are only B-cell deficient.


**Patentansprüche**

1. Verwendung von selektiven B-Zell-Depletionsmitteln für die Herstellung eines Medikaments für die Behandlung oder Vorbeugung von übertragbarer spongiformer Enzephalopathie in infizierten Menschen oder Tieren.

2. Verwendung gemäß Anspruch 1, **dadurch gekennzeichnet, dass** die B-Zell-Depletionsmittel immunologisch aktive Biomoleküle umfassen.

3. Verwendung gemäß Anspruch 2, **dadurch gekennzeichnet, dass** die immunologisch aktiven Biomoleküle Antikörper umfassen.

4. Verwendung gemäß Anspruch 3, **dadurch gekennzeichnet, dass** die Antikörper anti-$\mu$M-Antikörper umfassen.

5. Verwendung gemäß Anspruch 3, **dadurch gekennzeichnet, dass** die Antikörper LR1-Antikörper umfassen.

6. Verwendung gemäß Anspruch 3, **dadurch gekennzeichnet, dass** die Antikörper B220-Antikörper umfassen.

7. Verwendung gemäß Anspruch 1, **dadurch gekennzeichnet, dass** die B-Zell-Depletionsmittel immunsuppressiv aktive chemische Verbindungen umfassen.

8. Verwendung gemäß Anspruch 7, **dadurch gekennzeichnet, dass** die immunsuppressiv aktiven chemischen Verbindungen Ciamexon umfassen.

9. Verwendung gemäß Anspruch 7, **dadurch gekennzeichnet, dass** die immunsuppressiv aktiven chemischen Verbindungen Imexon umfassen.

10. Menschliches TSE-freies Körperflüssigkeits- oder Gewebe-abgeleitetes Produkt, **dadurch gekennzeichnet, dass** das Körperflüssigkeits- oder Gewebe-abgeleitete Produkt nur von B-Zellen in vitro depletiert wurde, indem es mit einem Medikament behandelt wurde, das ein selektives B-Zell-Depletionsmittel umfasst.

11. TSE-freies Körperflüssigkeits- oder Gewebe-abgeleitetes Produkt gemäß Anspruch 10, **dadurch gekennzeichnet, dass** das B-Zell-Depletionsmittel immunologisch aktive Biomoleküle umfasst.

12. TSE-freies Körperflüssigkeits- oder Gewebe-abgeleitetes Produkt gemäß Anspruch 11, **dadurch gekennzeichnet, dass** die immunologisch aktiven Biomoleküle Antikörper umfassen.

13. TSE-freies Körperflüssigkeits- oder Gewebe-abgeleitetes Produkt gemäß Anspruch 12, **dadurch gekennzeichnet, dass** die Antikörper anti-$\mu$M-Antikörper umfassen.

14. TSE-freies Körperflüssigkeits- oder Gewebe-abgeleitetes Produkt gemäß Anspruch 12, **dadurch gekennzeichnet, dass** die Antikörper LR1-Antikörper umfassen.

15. TSE-freies Körperflüssigkeits- oder Gewebe-abgeleitetes Produkt gemäß Anspruch 12, **dadurch gekennzeichnet, dass** die Antikörper B220-Antikörper umfassen.

16. TSE-freies Körperflüssigkeits- oder Gewebe-abgeleitetes Produkt gemäß Anspruch 10, **dadurch gekennzeichnet, dass** das B-Zell-Depletionsmittel immunsuppressiv aktive chemische Verbindungen umfasst.

17. TSE-freies Körperflüssigkeits- oder Gewebe-abgeleitetes Produkt gemäß Anspruch 16, **dadurch gekennzeichnet, dass** die immunsuppressiv aktiven chemischen Verbindungen Ciamexon umfassen.

18. TSE-freies Körperflüssigkeits- oder Gewebe-abgeleitetes Produkt gemäß Anspruch 16, **dadurch gekennzeichnet, dass** die immunsuppressiv aktiven chemischen Verbindungen Imexon umfassen.

19. Verfahren zur Herstellung eines menschlichen TSE-freien Körperflüssigkeits- oder Gewebe-abgeleiteten Produkts, **dadurch gekennzeichnet, dass** das Verfahren einen Schritt der Depletion von nur B-Zellen aus dem Körperflüssigkeits- oder Gewebe-abgeleiteten Produkt *in vitro* umfasst, worin die B-Zellen durch ein Medikament depletiert werden, das ein selektives B-Zell-Depletionsmittel umfasst.

20. Verfahren gemäß Anspruch 19, **dadurch gekennzeichnet, dass** das B-Zell-Depletionsmittel immunologisch aktive Biomoleküle umfasst.

21. Verfahren gemäß Anspruch 20, **dadurch gekennzeichnet, dass** die immunologisch aktiven Biomoleküle Antikörper umfassen.

22. Verfahren gemäß Anspruch 21, **dadurch gekennzeichnet, dass** die Antikörper anti-$\mu$M-Antikörper umfassen.

23. Verfahren gemäß Anspruch 21, **dadurch gekennzeichnet, dass** die Antikörper LR1-Antikörper umfassen.

24. Verfahren gemäß Anspruch 21, **dadurch gekennzeichnet, dass** die Antikörper B220-Antikörper umfassen.

25. Verfahren gemäß Anspruch 19, **dadurch gekennzeichnet, dass** das B-Zell-Depletionsmittel immunsuppressiv aktive chemische Verbindungen umfasst.

26. Verfahren gemäß Anspruch 25, **dadurch gekennzeichnet, dass** die immunsuppressiv aktiven chemischen Verbindungen Ciamexon umfassen.

27. Verfahren gemäß Anspruch 25, **dadurch gekennzeichnet, dass** die immunsuppressiv aktiven chemischen Verbindungen Imexon umfassen.

28. Verfahren zur Isolierung von menschlichen TSE-freien Körperflüssigkeits- oder Gewebe-abgeleiteten Produkten, **dadurch gekennzeichnet, dass** die Körperflüssigkeits- oder Gewebe-abgeleiteten Produkte aus nicht lebenden Menschen isoliert wurden, denen nur B-Zellen fehlen.

29. Menschliches TSE-freies Körperflüssigkeits- oder Gewebe-abgeleitetes Produkt, **dadurch gekennzeichnet, dass** das Körperflüssigkeits- oder Gewebe-abgeleitete Produkt aus Menschen isoliert wurde, denen nur B-Zellen fehlen.

**Revendications**

1. Utilisation d'agents de déplétion sélectifs des lymphocytes B pour fabriquer un médicament pour le traitement ou la prévention de l'encéphalopathie spongiforme transmissible chez des humains ou des animaux infectés.

2. Utilisation selon la revendication 1, **caractérisée en ce que** lesdits agents de déplétion des lymphocytes B comprennent des biomolécules actives sur le plan immunologique.

3. Utilisation selon la revendication 2, **caractérisée en ce que** lesdites biomolécules actives sur le plan immunologique comprennent des anticorps.

4. Utilisation selon la revendication 3, **caractérisée en ce que** lesdits anticorps comprennent des anticorps anti-$\mu$M.

5. Utilisation selon la revendication 3, **caractérisée en ce que** lesdits anticorps comprennent des anticorps LR1.

6. Utilisation selon la revendication 3, **caractérisée en ce que** lesdits anticorps comprennent des anticorps B220.

7. Utilisation selon la revendication 1, **caractérisée en ce que** lesdits agents de déplétion des lymphocytes B comprennent des composés chimiques ayant une action d'immunosuppression.

8. Utilisation selon la revendication 7, **caractérisée en ce que** lesdits composés chimiques ayant une action d'immunosuppression comprennent la ciamexone.

9. Utilisation selon la revendication 7, **caractérisée en ce que** lesdits composés chimiques ayant une action d'immunosuppression comprennent l'Imexon.

10. Produit humain dérivé de liquide organique ou de tissu exempts d'ESST, **caractérisé en ce que** ledit produit dérivé de liquide organique ou de tissu a été soumis à déplétion des lymphocytes B uniquement *in vitro,* par traitement avec un médicament comprenant un agent de déplétion sélectif des lymphocytes B.

11. Produit dérivé de liquide organique ou de tissu exempts d'ESST selon la revendication 10, **caractérisé en ce que** ledit agent de déplétion des lymphocytes B comprend des biomolécules actives sur le plan immunologique.

12. Produit dérivé de liquide organique ou de tissu exempts d'ESST selon la revendication 11, **caractérisé en ce que** lesdites biomolécules actives sur le plan immunologique comprennent des anticorps.

13. Produit dérivé de liquide organique ou de tissu exempts d'ESST selon la revendication 12, **caractérisé en ce que** lesdits anticorps comprennent des anticorps anti-$\mu$M.

14. Produit dérivé de liquide organique ou de tissu exempts d'ESST selon la revendication 12, **caractérisé en ce que** lesdits anticorps comprennent des anticorps LR1.

15. Produit dérivé de liquide organique ou de tissu exempts d'ESST selon la revendication 12, **caractérisé en ce que** lesdits anticorps comprennent des anticorps B220.

16. Produit dérivé de liquide organique ou de tissu exempts d'ESST selon la revendication 10, **caractérisé en ce que** ledit agent de déplétion des lymphocytes B comprend des composés chimiques ayant une action d'immunosuppression.

17. Produit dérivé de liquide organique ou de tissu exempts d'ESST selon la revendication 16, **caractérisé en ce que** lesdits composés chimiques ayant une action d'immunosuppression comprennent la ciamexone.

18. Produit dérivé de liquide organique ou de tissu exempts d'ESST selon la revendication 16, **caractérisé en ce que** lesdits composés chimiques ayant une action d'immunosuppression comprennent l'Imexon.

19. Procédé pour préparer un produit humain dérivé de liquide organique ou de tissu exempts d'ESST, **caractérisé en ce que** ledit procédé comprend une étape consistant en une déplétion des lymphocytes B uniquement dans ledit produit dérivé de liquide organique ou de tissu *in vitro,* la déplétion des lymphocytes B étant réalisée au moyen d'un médicament comprenant un agent sélectif de déplétion des lymphocytes B.

20. Procédé selon la revendication 19, **caractérisé en ce que** ledit agent de déplétion des lymphocytes B comprend des biomolécules actives sur le plan immunologique.

21. Procédé selon la revendication 20, **caractérisé en ce que** lesdites biomolécules actives sur le plan immunologique comprennent des anticorps.

22. Procédé selon la revendication 21, **caractérisé en ce que** lesdits anticorps comprennent des anticorps anti-$\mu$M.

23. Procédé selon la revendication 21, **caractérisé en ce que** lesdits anticorps comprennent des anticorps LR1.

24. Procédé selon la revendication 21, **caractérisé en ce que** lesdits anticorps comprennent des anticorps B220.

25. Procédé selon la revendication 19, **caractérisé en ce que** lesdits agents de déplétion des lymphocytes B comprennent des composés chimiques ayant une action d'immunosuppression.

26. Procédé selon la revendication 25, **caractérisé en ce que** lesdits composés chimiques ayant une action d'immunosuppression comprennent la ciamexone.

27. Procédé selon la revendication 25, **caractérisé en ce que** lesdits composés chimiques ayant une action d'immunosuppression comprennent l'Imexon.

28. Procédé pour isoler des produits humains dérivés de liquide organique ou de tissu exempts d'ESST, **caractérisé en ce que** lesdits produits dérivés de liquide organique ou de tissu sont isolés sur des humains non vivants qui sont déficients uniquement en lymphocytes B.

29. Produit humain dérivé de liquide organique ou de tissu exempt d'ESST, **caractérisé en ce que** ledit produit dérivé de liquide organique ou de tissu est isolé sur des humains qui sont déficients uniquement en lymphocytes B.

Fig. 1

Fig. 2a

Fig. 2b

Fig. 2c

Fig. 2d

Fig. 3a

Fig. 3b

Fig. 4

* B and T cells enriched or depleted
by magnetic activated cell sorting
(MACS) and complement lysis.

Wild type mice
"Spleen mice"

Fig. 5A

WILDTYPE MICE

Fig. 5B

"T CELL MICE"

Fig. 5C    "SPLEEN MICE"

MONTHS AFTER INOCULATION